# EUROPÄISCHE PATENTSCHRIFT

(11) **EP 1 670 826 B1**
(45) Veröffentlichungstag und Bekanntmachung des Hinweises auf die Patenterteilung: **29.02.2012**
(21) Anmeldenummer: 04765893.5
(22) Anmeldetag: 08.10.2004
(51) Int. Cl.: C07K 16/28, C12N 15/13, C12N 5/10, C12N 15/63, A61K 39/395, G01N 33/53

(54) **SINGLE-CHAIN-ANTIKÖRPER GEGEN DEN 37 KDA/67 KDA LAMININREZEPTOR ALS WERKZEUGE ZUR DIAGNOSE UND THERAPIE VON PRIONERKRANKUNGEN UND KREBS, DEREN HERSTELLUNG UND VERWENDUNG**
SINGLE-CHAIN ANTIBODY ACTING AGAINST 37 KDA/67 KDA LAMININ RECEPTOR AS TOOLS FOR THE DIAGNOSIS AND THERAPY OF PRION DISEASES AND CANCER, PRODUCTION AND USE THEREOF
ANTICORPS A CHAINE SIMPLE AGISSANT CONTRE LE RECEPTEUR DE LAMININE 37 KDA/67 KDA, UTILISES COMME OUTILS POUR DIAGNOSTIQUER ET TRAITER DES MALADIES A PRIONS ET DES CANCERS, LEUR MODE DE PRODUCTION ET LEUR UTILISATION

(30) Priorität: 08.10.2003 DE 10346627
(43) Veröffentlichungstag der Anmeldung: 21.06.2006
(73) Patentinhaber: Affimed Therapeutics AG, 69120 Heidelberg (DE); Ludwig-Maximilians-Universität München, 80333 München (DE)
(72) Erfinder: KNACKMUSS, Stefan, 68723 Plankstadt (DE); REY, Clémence, 34090 Montpellier (FR); RÖTTGEN, Peter, 68526 Ladenburg (DE); BÜTTNER, Claudia, 68723 Schwetzingen (DE); REUSCH, Uwe, 67487 Maikammer (DE); WEISS, Stefan, 2050 Wits, ZA Johannesburg (ZA); LITTLE, Melvyn, 25826 St. Peter-Ording (DE)
(74) Vertreter: Schüssler, Andrea
(86) Internationale Anmeldenummer: PCT/EP2004/011268
(87) Internationale Veröffentlichungsnummer: WO 2005/035580

(56) Entgegenhaltungen:
- EP-A1- 1 127 894
- WO-A1-89/11273
- US-A- 4 686 180
- GESUNDHEITSFORSCHUNG DES BMBF: "Vorhabenübersicht: TSE-Neurodegenerative Erkrankugen" BEKANNTMACHUNG DES BUNDESMINISTERIUM FUER BILDUNG UND FORSCHUNG, [Online] 2001, Seiten 1-15, XP002316009 Gefunden im Internet: URL:http://www.gesundheitsforschung-bmbf.d e/foerderung/Vorhabenuebersicht/1-Bekaempf ung-Krankheiten/239-TSE/s_druck> [gefunden am 2005-02-02]
- RIEGER R ETAL: "The human 37-kDa laminin receptor precursor interacts with the prion protein in eukaryotic cells" NATURE MEDICINE, NATURE PUBLISHING, CO, US, Bd. 3, Nr. 12, Dezember 1997 (1997-12), Seiten 1383-1388, XP002094757 ISSN: 1078-8956
- COGGIN J H JR ET AL: "37 Kilodalton oncofetal antigen protein and immature laminin receptor protein are identical, universal T-cell inducing immunogens on primary rodent and human cancers" ANTICANCER RESEARCH, HELENIC ANTICANCER INSTITUTE, ATHENS,, GR, Bd. 19, Nr. 6C, November 1999 (1999-11), Seiten 5535-5542, XP002981891 ISSN: 0250-7005
- LITTLE M ET AL: "Generation of a large complex antibody library from multiple donors" JOURNAL OF IMMUNOLOGICAL METHODS, ELSEVIER SCIENCE PUBLISHERS B.V.,AMSTERDAM, NL, Bd. 231, Nr. 1-2, 10. Dezember 1999 (1999-12-10), Seiten 3-9, XP004187630 ISSN: 0022-1759
- HUTCHINGS, CARMENT, LENNARD: "Antibody engineering, Chapter 6, Generation of Naive Antibody Libraries." 2001, SPRINGER VERLAG BERLIN , BERLIN , XP002316010 ISBN: 3-540-41354-5 Seite 94 - Seite 108

## Beschreibung

Die vorliegende Erfindung liegt auf dem Gebiet von Antikörpern, welche gegen den Zelloberflächenrezeptor von Prionproteinen, den 37 kDa/67 kDa Lamininrezeptor, gerichtet sind. Insbesondere betrifft die vorliegende Erfindung Einzelketten-Antikörper, sog. "Single-chain"-Antikörper, die durch die Ansprüche definiert sind die spezifisch sowohl die 37 kDa Vorläuferform des Lamininrezeptors (37 kDa LRP) als auch die 67 kDa high-affinity-Form des Lamininrezeptors (67 kDa LR) erkennen.

Prionproteine, die am Auftreten der verschiedensten Formen von übertragbaren spongiformen Encephalopathien (TSEs) beteiligt sind, wie Scrapie bei Schafen, Mäusen und Hamstern, übertragbare spongiforme Encephalopathie von Rindern (BSE), übertragbare spongiforme Encephalopathie bei Nerzen (TME), Kuru, Gerstmann-Sträußler-Scheinker-Syndrom (GSS), Creutzfeldt-Jakob-Krankheit (CJD) und fatale familiäre Insomnie (FFI) beim Menschen, bestehen hauptsächlich, wenn auch nicht gänzlich aus PrP^{Sc}, einer anomalen Isoform des ubiquitären zellulären Prionenproteins PrP^{c} (siehe beispielsweise Aguzzi and Weissmann, 1998; Lasmézas and Weiss, 2000; Prusiner et al., 1998).

Aus der WO 98/53838 sowie aus Rieger et al. (Rieger et al., 1997) ist bekannt, dass das PrP^{c} spezifisch an den Zelloberflächenrezeptor von Prionproteinen, den 37 kDa LRP/67 kDa LR, bindet. Aus der WO 98/53838 sowie der EP-A-1 127 894 und aus Rieger et al., (Rieger et al., 1997) ist bekannt, dass der Lamininrezeptorspiegel in Geweben von mit Scrapie infizierten Tieren, wie Hamster und Mäusen, erhöht ist.

Gauczynski et al., 2001b zeigen, dass der 37 kDa LRP/67 kDa LR als Rezeptor für PrP^{c} fungiert. Bindestellen auf beiden Molekülen wurden gemappt (Hundt et al., 2001) und Heparansulfatproteoglykane (HSPGs) als Cofaktoren bzw. Corezeptoren für PrP^{c} identifiziert (Hundt et al., 2001).

Der 37 kDa *laminin receptor precursor* (37 kDa LRP, p40, LBP) ist das Vorläuferprotein des 67 kDa *high affinity* Lamininrezeptors (67 kDa LR) (Rao et al., 1989; Yow et al., 1988). Die 67 kDa Form wurde zunächst aus Tumorzellen isoliert (Lesot et al., 1983; Malinoff and Wicha, 1983; Rao et al., 1983), wo das Protein eine hohe Affinität zu Laminin ausweist.

Der 37 kDa/67 kDa Lamininrezeptor ist ferner in Tumorgeweben überexprimiert (Lesot et al., 1983; Malinoff and Wicha, 1983; Rao et al., 1983).

Laminin ist ein Glykoprotein der extrazellulären Matrix, wo es an der Anhaftung, Bewegung, Differenzierung und Wachstum von Zellen beteiligt ist (Beck et al., 1990). Beide Formen des Lamininrezeptors existieren nebeneinander in Säugerzellen, was durch immunologische Studien von Membranfraktionen gezeigt werden konnte (Gauczynski et al., 2001b).

Die 37 kDa Form kommt auch im Zytosol vor, wo sie mit Ribosomen assoziiert ist und Aufgaben bei der Proteintranslation übernehmen kann (Auth and Brawerman, 1992; Sato et al., 1999). Es wurde auch die Existenz dieses Proteins im Kern diskutiert, wo sie bei der Aufrechterhaltung von Strukturen involviert sein soll (Kinoshita et al., 1998; Sato et al., 1996).

Bei LRP/LR handelt es sich um ein multifunktionelles Protein, welches ausgehend von dem Genprodukt p40 zwei unterschiedliche Formen bilden kann, in verschiedenen Zellkompartimenten vorkommt und dort unterschiedliche Funktionen ausübt. Die Aminosäuresequenz des 37 kDa/67 kDa Lamininrezeptors ist hochkonserviert, mit einer hohen Homologie bei Säugern (Rao et al., 1989). Durch eine Evolutionsanalyse der Aminosäuresequenz konnte gezeigt werden, dass die palindromische Sequenz LMWWML verantwortlich für die Fähigkeit ist, Laminin zu binden. Diese Sequenz liegt im PrPbindenden Bereich des 37 kDa LRP (Ardini et al., 1998; Hundt et al., 2001; Rieger et al., 1997).

Es scheint, dass das ribosomale Protein p40, das zunächst nicht die Fähigkeit besaß Laminin zu binden (Auth and Brawerman, 1992), im Laufe der Evolution durch Aminosäureaustausch und Einführung won posttranslationalen Veränderungen zu einem lamininbindenden Zelloberflächenprotein evolvierte, welches auch Elastin (Hinek et al., 1988; Salas et al., 1992) und Kohlenhydratketten (siehe beispielsweise Ardini et al., 1998; Mecham, 1991; Rieger et al., 1999) binden kann.

Die Lamininrezeptorfamilie ist in vielen eukaryotischen Zellen hochkonserviert (Keppel and Schaller, 1991; Wewer et al., 1986) und kann auch in *Archaea* gefunden werden (Ouzonis et al., 1995). Der 37 kDa LRP fungiert als Rezeptor für das Venezuelanische Equine Enzephalitis Virus auf Moskitozellen (Ludwig et al., 1996), während die 67 kDa Form offensichtlich als Rezeptor für das Sindbis Virus dienen kann (Wang et al., 1992).

Hinsichtlich der der Umwandlung der 37 kDa Form in das 67 kDa Protein zugrundeliegenden Prozesse ist bekannt, dass beide Proteine aus der 37 kDa Komponente bestehen, wobei mehrere Vorschläge gemacht wurden, die größere Masse-des reifen Proteins zu erklären. Homodimerisierung des 37 kDa Proteins, wie auch die Bindung einer anderen Komponente wurden diskutiert (Castronovo et al., 1991; Landowski et al., 1995). Andere Studien jedoch schlagen ein durch Fettsäuren stabilisiertes Heterodimer vor (Buto et al., 1998). Kürzlich wurde gezeigt, dass der 67 kDa Lamininrezeptor auch auf aktivierten humanen T-Lymphozyten vorkommt und dort zusammen mit Integrinen eine starke Affinität zu Laminin aufweist (Canfield and Khakoo, 1999).

Es ist jedoch festzuhalten, dass gegenwärtig der 37 kDa/67 kDa Polymorphismus nicht abschließend geklärt ist.

Der 37 kDa/67 kDa Lamininrezeptor ist durch mehrere Gene im Genom von Säugern vertreten. Beim Menschen sind es 26, bei der Maus 6 Kopien (Fernandez et al., 1991; Jackers et al., 1996b). Das Gen besteht aus sieben Exons und sechs Introns, wobei es sich bei den meisten Genkopien wahrscheinlich um Pseudogene handelt (Jackers et al., 1996a). Bei der Maus gibt es Hinweise, dass mindestens zwei der sechs Gene aktiv sind und sich auf Chromosom 9 befinden (Douville and Carbonetto, 1992; Fernandez et al., 1991). Mit Hilfe von TRIBE-MCL, ein Algorithmus zur Detektion von Proteinfamilien (Enright et al., 2002), wurden fünf LRP-Gene bei der Verwendung des Programms zum Screening der aktuellen Maus Genom Sequenz Datenbank (www..ensembl.org) identifiziert. Interessanterweise wurde kürzlich gezeigt, dass sich auf Chromosom 9 möglicherweise Genloci befinden, welche die Inkubationszeit von Prionenerkrankungen bei Mäusen beeinflussen (Stephenson et al., 2000).

Das Gen, das für den 37 kDa LRP codiert, ist in vielen verschiedenen Spezies identifiziert worden, wie *Saccharomyces cerevisiae* (Davis et al., 1992), *Arabidopsis thaliana* (Garcia-Hemandez et al., 1994), *Drosophila melanogaster* (Melnick et al., 1993), dem Seeigel *Urechis caupo* (Rosenthal and Wordeman, 1995), *Chlorohydra veridissima* (Keppel and Schaller, 1991), *Candida albicans* (Lopez-Ribot et al., 1994) und dem Archaebakterium *Haloarcula marismortui* (Ouzonis et al., 1995) und den Säugern (siehe beispielsweise Gauczynski et al., 2001a; Leucht and Weiss, 2002; Rieger et al., 1999).

Aus der WO 98/53838 sowie aus einer späteren Publikation (Leucht et al., 2003) ist bekannt, dass der polyklonale LRP-Antikörper W3 die PrP^{Sc}-Vermehrung in kultivierten neuronalen Zellen verhindert. Dies zeigt, dass generell Antikörper gegen 37kDa LRP/67 kDa LR die Prionenvermehrung zumindest in Zellkultur vollständig unterdrücken können. Interessanterweise blieben die Zellen auch dann frei von PrP^{Sc} (Leucht et al., 2003), wenn nach Weiterkultivierung für weitere zwei Wochen kein LRP/LR-Antikörper zugegeben wurde. Dies zeigt, dass generell LRP/LR-Antikörper in der Lage sind, Prion-infizierte Zellkulturen von einer Prioninfektion vollständig zu heilen.

Weiterhin ist aus der EP-A-1 127 894 bekannt, dass LRP/LR-Antikörper diagnostisch zur Erkennung von Prionerkrankungen eingesetzt werden können, da der LRP/LR-Spiegel in Geweben von mit Scrapie infizierten Nagern erhöht ist.

Polyklonale Antikörper gegen LRP/LR, die in der WO 98/53838 und der EP-A-1 127 894 beschrieben sind, besitzen jedoch den Nachteil, dass sie nur schwierig zu selektieren sind, sehr groß sind und eine hohe Immunogenität zeigen. Diese Antikörper stehen im Gegensatz zu Single-chain-Antikörpern, welche in grossen Mengen in E.coli synthetisiert werden können, nur in begrenztem Umfang zur Verfügung.

Aufgabe der vorliegenden Erfindung war es folglich, neue monoklonale Antikörper, welche spezifisch LRP/LR erkennen, bereitzustellen, die leicht zu selektieren sind, sich durch eine geringe Größe auszeichnen und eine geringe Immunogenität aufweisen.

Gegenstand eines ersten Aspekts der vorliegenden Erfindung ist ein Single-chain-Antikörpermolekül, welches spezifisch gegen LRP/LR gerichtet ist und das die Aminosäuresequenz SEQ ID Nr.2 umfasst.

Gegenstand eines weiteren Aspekts der vorliegenden Erfindung ist ein Single-chain-Antikörpermolekül, welches spezifisch gegen LRP/LR gerichtet ist und das die Aminosäuresequenz SEQ ID Nr. 4 umfasst.

Gegenstand eines weiteren Aspekts der vorliegenden Erfindung ist eine cDNA, die für das Antikörpermolekül mit der Bezeichnung S 18 codiert, welches gegen LRP/LR gerichtet ist und das die Nukleotidsequenz SEQ ID Nr.1 umfasst.

Gegenstand eines weiteren Aspekts der vorliegenden Erfindung ist eine cDNA, die für das Antikörpermolekül mit der Bezeichnung N3 codiert, welches gegen LRP/LR gerichtet ist und das die Nukleotidsequenz SEQ ID Nr.3 umfasst.

Gegenstand eines weiteren Aspekts der vorliegenden Erfindung ist ein Replikations- oder Expressionvektor, der die erfindungsgemäße cDNA trägt. Die Vektoren können beispielsweise Plasmid-, Virus- oder Phagenvektoren sein, die einen Replikationsursprung und gegebenenfalls einen Promotor für die Expression der cDNA und gegebenenfalls einen Regulator des Promotors umfassen. Der Vektor kann ein oder mehrere selektierbare Markergene, beispielsweise das Ampicillinresistenzgen, enthalten. Der Vektor kann in vitro, beispielsweise zur Produktion von RNA entsprechend der cDNA oder zumTransfizieren einer Wirtszelle verwendet werden. Als Vektoren eignen sich beispielsweise virale Vektoren (Lentiviren, Adenoviren, Adeno-associated Viren (AAV)).

Gegenstand eines weiteren Aspekts der vorliegenden Erfmdung sind Wirtszellen, die mit den Vektoren für die Replikation und Expression der erfindungsgemäßen cDNA, einschließlich der cDNA, die die Nukleotidsequenz SEQ ID Nr. 1 oder 3 oder den offenen Leserahmen hiervon umfasst, transformiert sind. Die Zellen sind so gewählt, dass sie mit dem Vektor kompatibel sind. Beispiele hierfür sind Bakterien-, Hefe-, Insektenzellen oder Säugerzellen, insbesondere E.coli-Zellen oder Säugerzellen.

Gegenstand eines weiteren Aspekts der vorliegenden Erfindung ist ein Verfahren zur Herstellung eines Antikörpermoleküls, das ein Kultivieren von Wirtszellen gemäß der vorliegenden Erfindung unter zur Expression eines erfindungsgemäßen Antikörpermoleküls wirksamen Bedingungen umfasst.

Gegenstand eines weiteren Aspekts der vorliegenden Erfindung sind pharmazeutische Zusammensetzungen, die ein erfindungsgemäßes Antikörpermolekül in Verbindung mit einem pharmazeutisch akzeptablen Verdünnungsmittel und/oder Träger umfassen.

In einer weiteren bevorzugten Ausführungsform eignet sich die oben genannte pharmazeutische Zusammensetzung zur Behandlung von Prionerkrankungen.

Gegenstand eines weiteren Aspekts der vorliegenden Erfindung sind diagnostische Zusammensetzungen, die ein erfindungsgemäßes Antikörpermolekül in Verbindung mit einem akzeptablen Verdünnungsmittel und/oder Träger umfassen.

Das oben beschriebene Antikörpermolekül umfasst auch solche, bei denen ein Teil ihrer Aminosäuresequenz fehlt (d.h. ein Antikörpermolekül, das lediglich die essentielle Sequenz zur Entfaltung der biologischen Aktivität der in SEQ ID Nr. 2 oder 4 angegebenen Aminosäuresequenz umfasst), solche, bei denen ein Teil ihrer Aminosäuresequenz durch andere Aminosäuren ersetzt ist (d.h. bei denen eine Aminosäure durch eine Aminosäure ähnlicher Eigenschaft ersetzt ist) und solche, bei denen andere Aminosäuren an bzw. in einen Teil ihrer Aminosäuresequenz addiert bzw. inseriert sind.

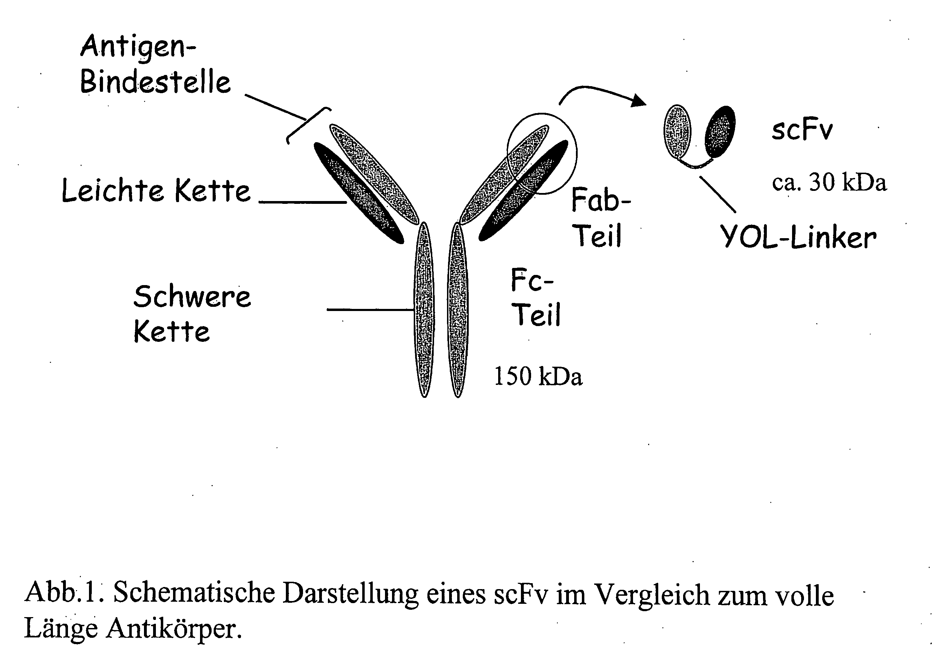

Abb. 1 zeigt die schematische Darstellung eines single chain (Einzelkettenantikörpers) scFv im Vergleich zum die volle Länge aufweisenden Antikörper, bestehend aus Fab- u. Fc-Teil. Der scFv besteht aus einem Teil der schweren Kette (V_{H}). und einem Teil der leichten Kette (V_{L}). Beide Teile stammen aus dem Fab Teil eines Antikörpers, welche für die Antigenerkennung zuständig ist. V_{H} und V_{L} sind durch einen Linker (YOL) verbunden.

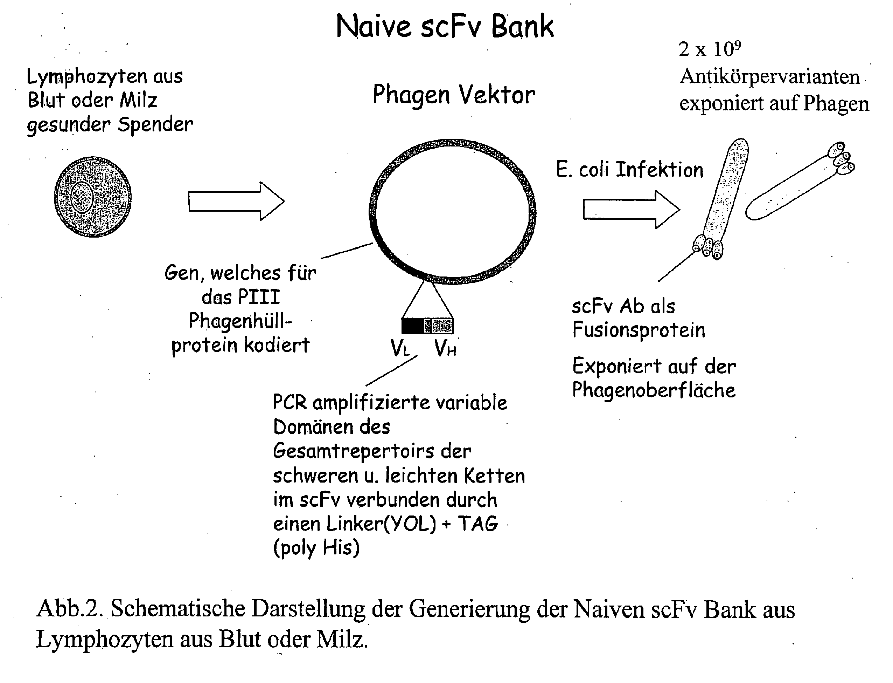

Abb. 2 zeigt schematisch die naïve scFv Bank, welche zum Screening der scFv Antikörper gerichtet gegen GST::LRP verwendet wurde. Die naïve Bank enthält etwa 2 x 10⁹ Klone und wurde durch die Kombination der kodierenden Regionen für die schweren (V_{H}) und leichten Ketten (V_{L}) nach PCR Amplifikation der respektiven cDNA aus Milz oder PBLs (Periphere Blutlymphozyten) generiert.

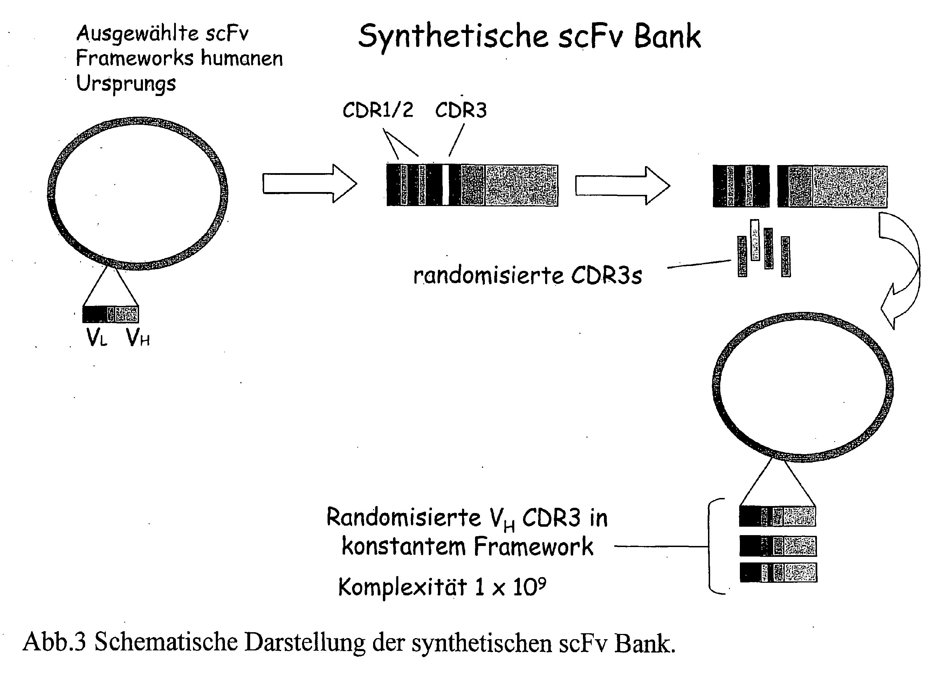

Abb. 3 zeigt schematisch die synthetische scFv Bank bestehend aus selektierten Frameworks mit guten Faltungseigenschaften und hohen Expressionsraten und enthaltend randomisierte Sequenzen in ihren CDR3 Regionen. Die Bank enthält etwa 1 x 10⁹ Klone.

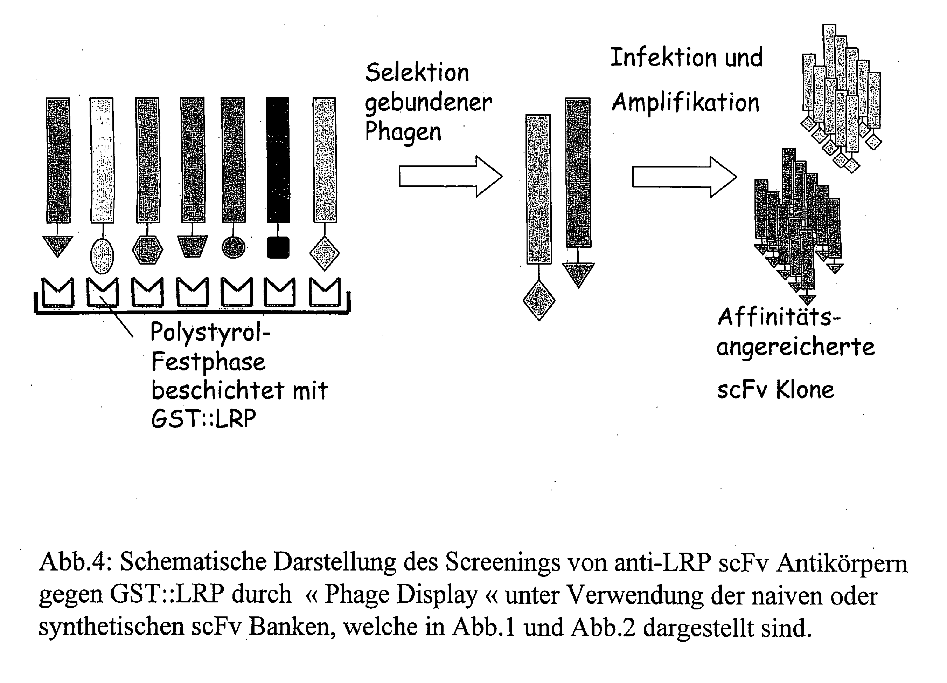

Abb. 4 zeigt schematisch das Screening-Verfahren über Phage-Display. Bei diesem Verfahren werden die in Abb. 2 und Abb. 3 dargestellten naiven oder synthetischen scFv Banken eingesetzt. Das in der WO 98/53838 beschriebene Antigen GST::LRP wurde auf Polystyrol-Tubes immobilisiert. Nur Phagen, welche scFv präsentieren, welche GST::LRP binden, werden selektiert. Unspezifisch gebundene Phagen werden weggewaschen. Es folgt eine Amplifikation dieser Phagen, wobei diese über drei aufeinanderfolgende Selektionsrunden angereichert werden.

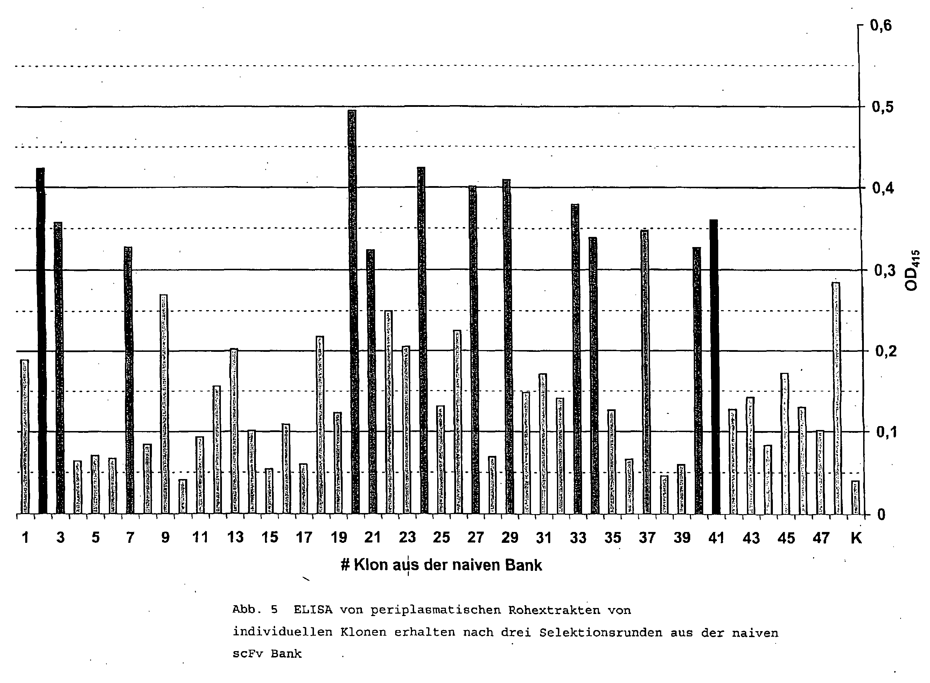

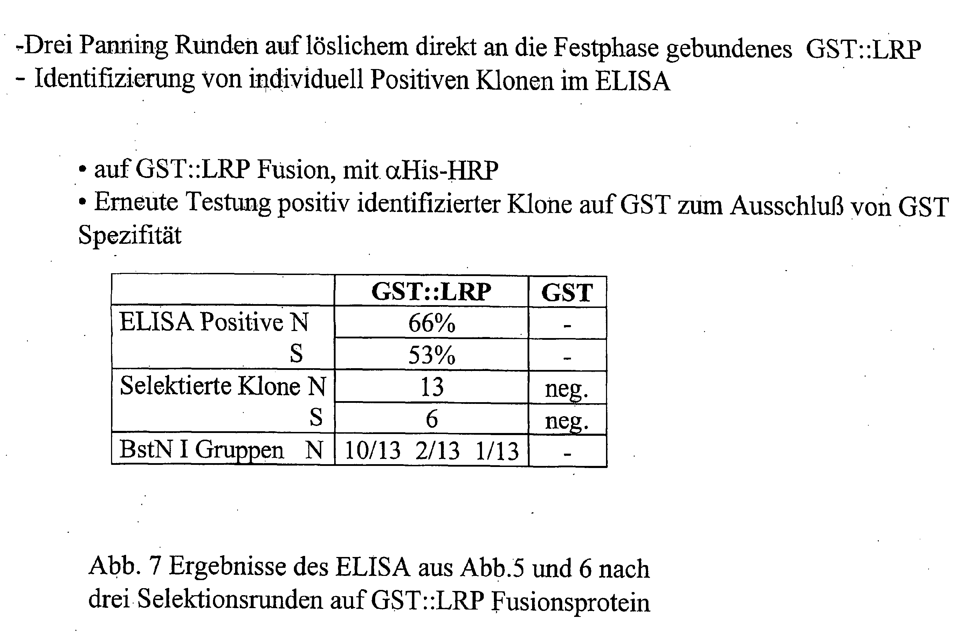

Abb. 5 zeigt einen ELISA von periplasmatischen. Rohextrakten von Klonen erhalten nach drei Selektionsrunden aus der naiven scFv Bank. Die Extrakte wurden an rekombinanten GST::LRP Fusionsprotein getestet. Das Ergebnis ist in Abb. 7 tabellarisch dargestellt. K = nur Sekundärantikörper.

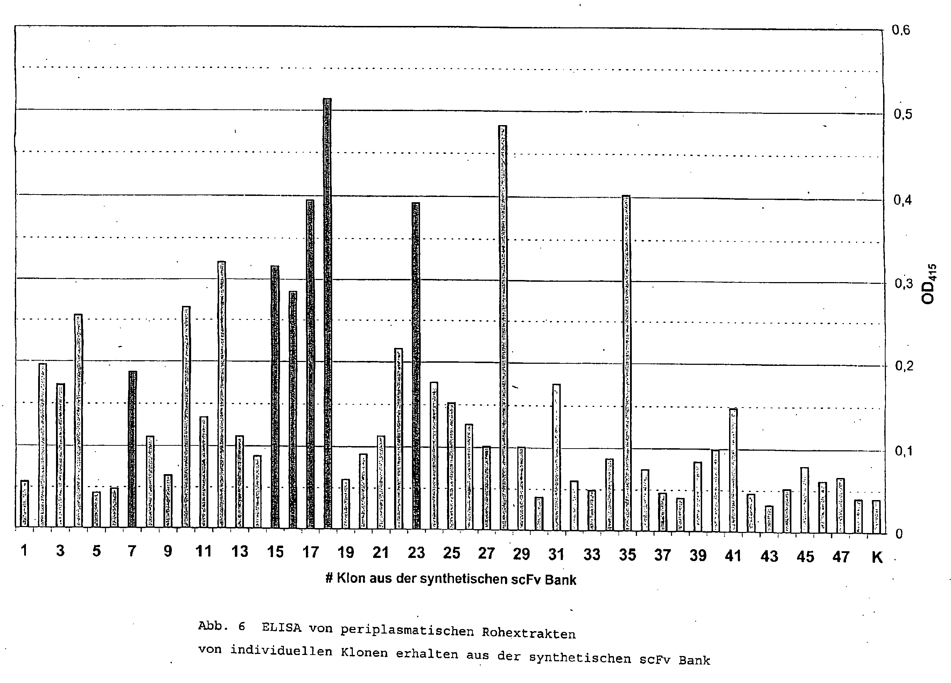

Abb. 6 zeigt einen ELISA von periplasmatischen Rohextrakten von Klonen erhalten nach drei Selektionsrunden aus der synthetischen scFv Bank. Die Extrakte wurden an rekombinanten GST::LRP Fusionsprotein getestet. Das Ergebnis ist in Abb. 7 tabellarisch-dargestellt. K = nur Sekundärantikörper.

Abb. 7 stellt das Ergebnis der ELISAs aus Abb. 5 und 6 schematisch zusammen. 32 aus 48 Klonen (66%) im Falle der naiven Bank (Abb. 5) und 25 aus 47 Klonen (53%) aus der synthetischen Bank zeigten positive Signale. Es wurde weiterhin eine Retestung von 13 Klonen aus der naiven und 6 Klonen aus der synthetischen Bank auf GST (ohne Fusionspartner) durchgeführt. GST wurde nicht erkannt, was darauf schliessen lässt, dass die scFvs nur den LRP-Teil des Fusionsproteins erkennen. Eine Restriktionsanalyse der cDNAs der 13 Klone aus der naiven Bank mit *Bst*NI zeigte, dass 10 Klone identisch waren (10/13). Ein weiterer Klon wurde zweimal identifiziert (2/13). Ein Klon (N37) ergab ein individuelles Restriktionsmuster.

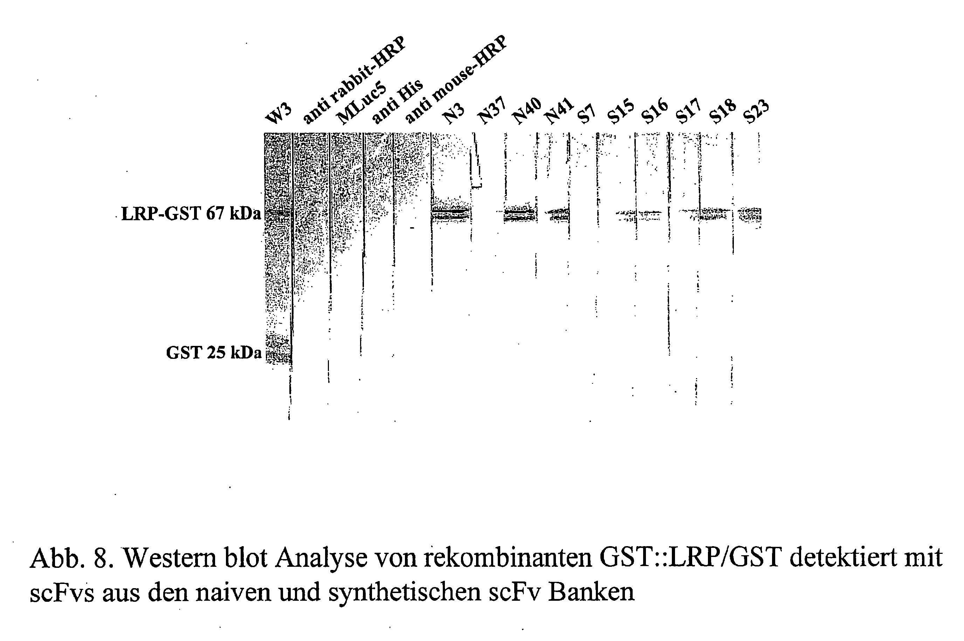

Abb.8 zeigt die Detektion von rekombinantem GST::LRP durch individuelle Klone selektiert aus der synthetischen (S) und naiven (N) scFv Bank. Ausgewählte Klone, welche über ELISA identifiziert wurden (Abb.5 u.6) wurden im Western Blot getestet. In jeder Spur wurde eine Mischung von rec. GST und GST::LRP exprimiert im Baculovirussystem auf einem 12%igen SDS-PA-Gel aufgetrennt. Die Proteine wurden auf eine Nitrozellulose-Membran geblottet. Die einzelnen Spuren des Blots wurden ausgeschnitten und die einzelnen Streifen mit periplasmatischen Extrakten der selektierten individuellen scFvs aus der naiven und synthetischen Bank oder respektiven Kontrollen inkubiert. Das polyklonale anti-LRP Antiserum W3 bekannt aus WO 98/53838 wurde mit einem polyklonalen Ziege anti-Rabbit (Hase) HRP-Konjugat (Dianova) detektiert. Die scFvs wurden mit einem monoklonalem Maus-anti-penta-Histidin Antikörper (Qiagen) gefolgt von einem Ziege Anti-Maus HRP-Konjugat (Dianova) detektiert. Anti-rabbit-HRP: nur Ziege-anti-Hase Konjugat. Anti-His: monoklonalerMaus anti Histidine Antikörper mit einem weiteren Ziege Anti Maus HRP Konjugat. Anti-Maus-HRP: nur Ziege anti-Maus-HRP Konjugat. MluC5: monoklonaler anti-LRP Antikörper, welcher in der Western Blot Analyse nicht funktioniert.

Die scFvs N3, sowie S 18 und S23 lieferten starke Signale. Keiner der scFv Antikörper erkannte rec. GST, was zeigt, dass alle scFvs den LRP Teil erkennen.

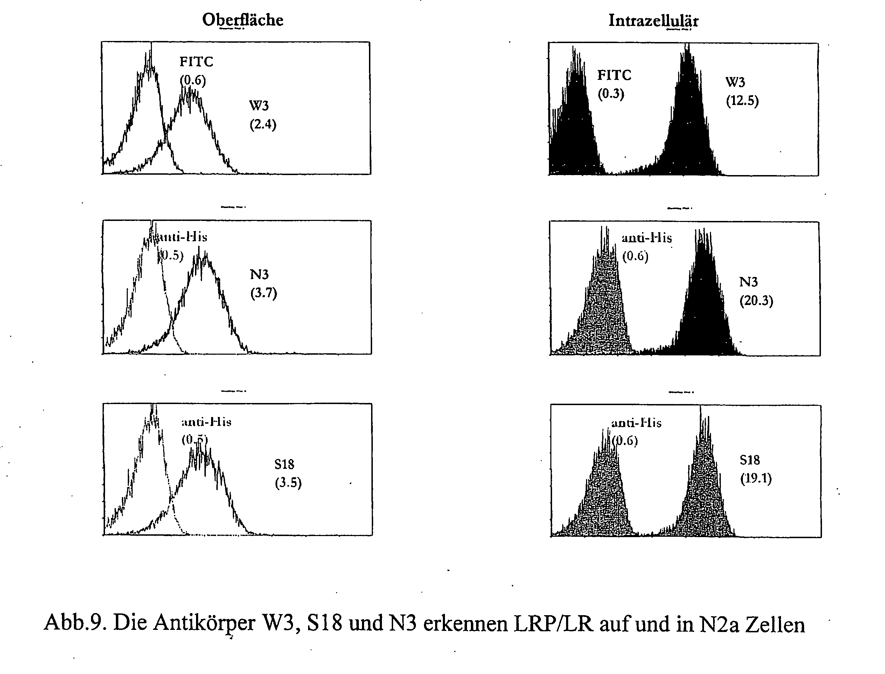

Abb.9 zeigt den Nachweis von LRP/LR auf der Oberfläche und intrazellulär von N2a Zellen. Oberfläche: Die Zellen wurden inkubiert mit 100 µg/ml anti-LRP Antiserum W3 (WO 98/53838), dann mit dem FTTC-konjugierten Ziege-anti-Hase IgG.
scFv anti-LR/LRP N3 und S18 wurden mit jeweils 18 µg/ml eingesetzt und detektiert mit monoklonalem anti-His-Antikörper (1:20;Dia900) gefolgt von FITC-konjugiertem Ziege-anti-Maus IgG.
Intrazellulär: Die Zellen wurden fixiert mit 3% Paraformaldehyd, und inkubiert mit 50 mM NH₄Cl/20 mM Glycin vor der Anfärbung mit den oben beschriebenen Antikörpern mit folgender Veränderung: alle Wasch- und Inkubationsschritte wurden bei Raumtemperatur in einem 0.1 % Saponin haltigen Puffer ausgeführt. Die scFv-Antikörper wurden jeweils zu Konzentrationen 9 µg/ml eingesetzt.

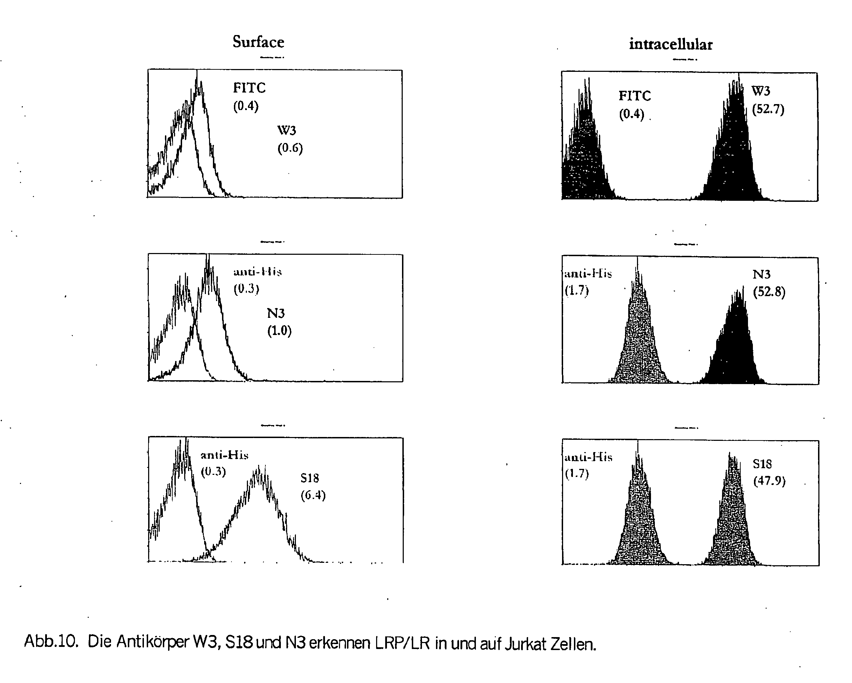

Abb. 10 zeigt den Nachweis von LRP/LR auf der Oberfläche und intrazellulär von Jurkat Zellen (humane, periphere Blutleukämie-T-Zellen). Die verwendeteten Antikörper, Sekundär und Tertiärantikörper entsprechen den in der Legende von Abb. 9 beschriebenen. Die verwendete Methode entspricht der in Abb. 9 beschriebenen ausser, dass Jurkat-Zellen verwendet wurden.

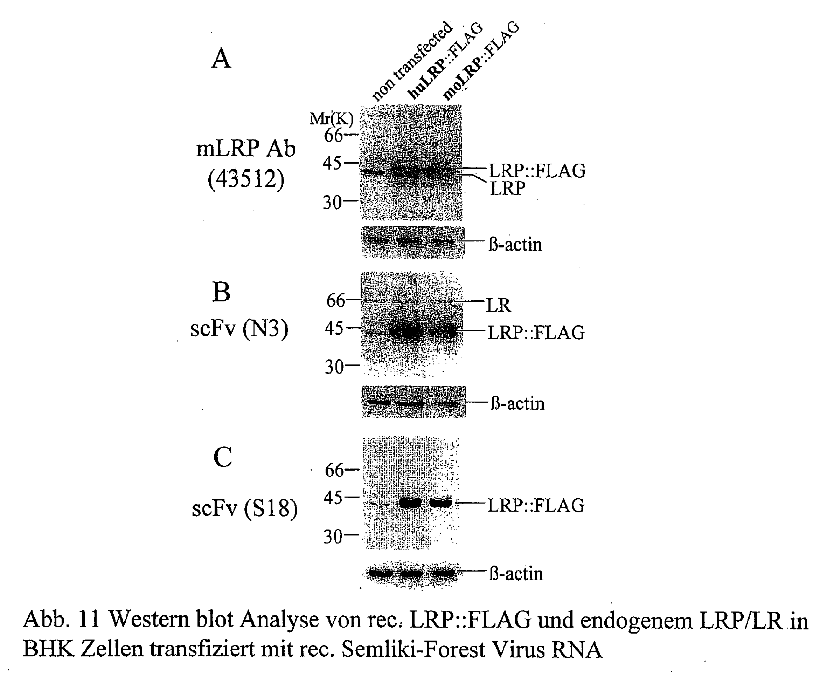

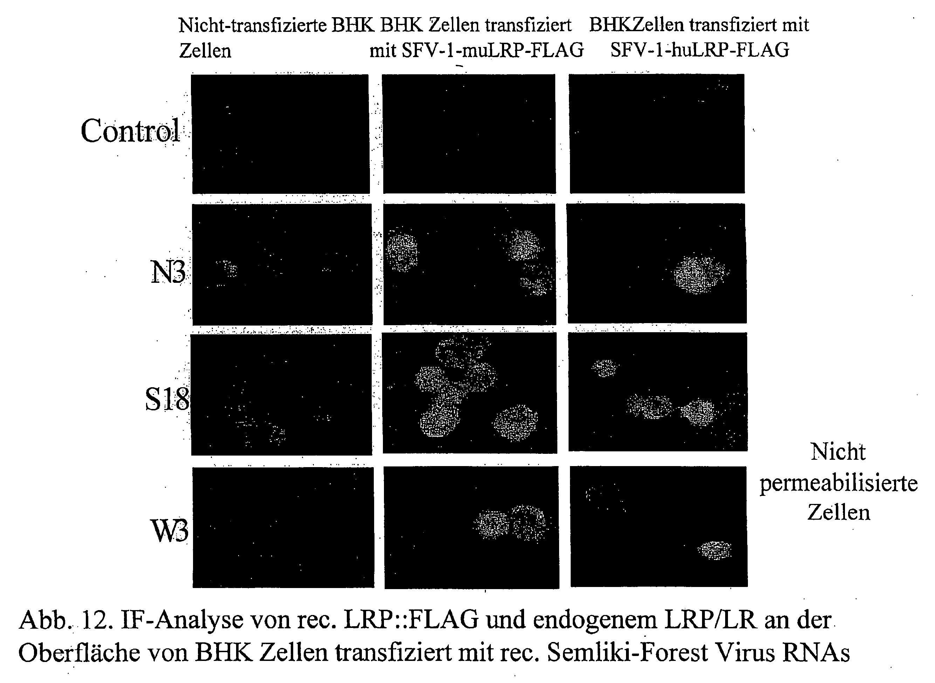

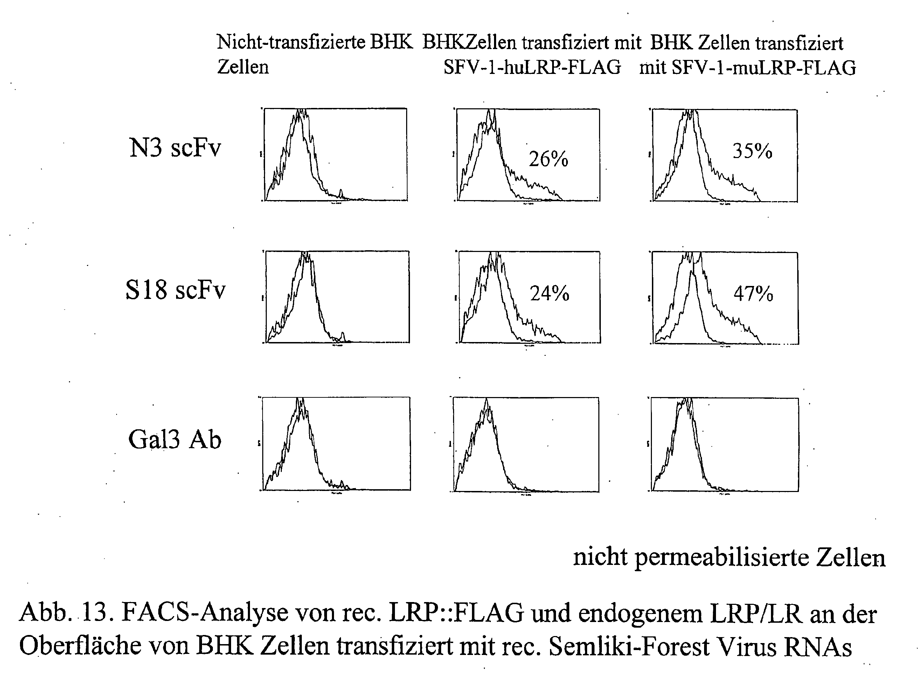

Abb. 11 zeigt den Nachweis von rekombinantem LRP::FLAG bzw. endogenem LRP/LR in Baby-Hamster Kidney Zellen (BHK) transfiziert mit SFV1-huLRP-FLAG bzw. SFV1-mo-LRP::FLAG. BHK-Zellen wurden auf dem Fachmann auf dem Fachgebiet bekannte Weise entweder nicht (n.t.) oder mit den rek. Semliki-Forest-Virus (SFV) RNAs SFV1-huLRP-FLAG bzw. SFV1-moLRP::FLAG transfiziert. Die Methode der Herstellung rec. SFV RNA, der Transfektion sowie der Analyse durch Western Blotting oder Immunofluoreszenz (Abb.12) oder FACS (Abb.13) wurden bereits beschrieben (Gauczynski et al., 2002; Gauczynski et al., 2001b). 24 h post Transfektion wurden Gesamtzellextrakte durch Western Blotting analysiert. Die Gesamtzellextrakte wurden auf einem 12 % igen PA-Gel aufgetrennt, danach die Proteine auf PVDF-Membran geblottet. Als Primärantikörper wurden die scFvs S18 (C) und N3 (A) (Verdünnung jeweils 1:1000) eingesetzt, als Sekundärantikörper Maus-anti-c-myc (1:1000) als Tertiärantikörper anti-Maus-HRP (horse raddish-peroxidase) gekoppelt. Als Kontrolle wurde der anti-LRP Antikörper mLRP43512 (A), sowie eine antimouse-IgG-HRP gekoppelter Sekundärantikörper verwendet. Als Ladungskontrolle wurde der ß-Actin Spiegel bestimmt. Dazu wurde ein anti-ß-actin Antikörper (Chemicon) eingesetzt. Nachweis erfolgte durch Chemiluminescenz (Westem Lightning, NEN). Die svFvS18 und N3 erkennen spezifisch das rec. LRP::FLAG. S18 und N3 erkennen endogenes LRP (B,C). N3 erkennt endogenen LR (67 kDa) (B).

Abb. 12. Immunofluoreszenz-Analyse von LRP/LR in BHK Zellen transfiziert mit rekombinater SFV RNA. BHK-Zellen wurden wie in Abb. 11 beschrieben nicht-transfiziert oder mit den rec. SFV RNAs SFV-1-muLRP-FLAG und SFV-1-huLRP-FLAG transfiziert. Die scFv Antikörper S18 und N3 sowie der aus der WO 98/53838 bekannte W3 Antikörper wurden jeweils 1: 100 eingesetzt. Für S18 und N3 wurde ein Anti-c-myc-FITC gekoppelter Sekundärantikörper jeweils 1:500 eingesetzt. Für W3 wurde ein anti-rabbit-Cy2 gekoppelter Antikörper (1:500) eingesetzt. Als Kontrolle wurde nur der Sekundärantikörper anti-c-myc-FITC (1:500) eingesetzt. Der Nucleus wurde mit DAPI gefärbt. Diese Färbung ist in der Abb. nicht zu sehen. Zellen wurden mit 4% Paraformaldehyd fixiert. Die Zellen wurden nicht permeabilisiert, was Zelloberflächen-Färbung garantiert. Die Abb. zeigt, dass die scFv S18 und N3 LRP::FLAG an der Oberfläche der transfizierten Zellen detektieren. Beide scFv S18 und N3 sind auch in der Lage endogenes LRP zu detektieren (2. u. 3. Linkes Bild von oben).

Abb. 13 zeigt ein FACS-Analyse von BHK-Zellen, welche nicht transfiziert oder mit den rekombinanten SFV-RNAs SFV-1-huLRP-FLAG bzw. SFV-1-muLRP-FLAG transfiziert wurden. ScFVS18 und N3 wurden in der Konz. 18 µg/ml, der W3 Antikörper und der Anti-Galectin-3 Antikörper mit 100 µg/ml eingesetzt. Sekundärantikörper: anti-c-myc-FITC gekoppelt für S 18 und N3, anti-rabbit-Cy2 für W3 und anti-Maus-Cy2 gekoppelt für anti-gal3 Antikörper (Verdünnung der Sekundärantikörper 1:500). Zellen wurden nicht permeabilisiert, was Zelloberflächenfärbung garantiert. Die Abbildung zeigt, dass scFv S18 und N3 LRP::FLAG auf der Zelloberfläche lebender Zellen nachweisen können.

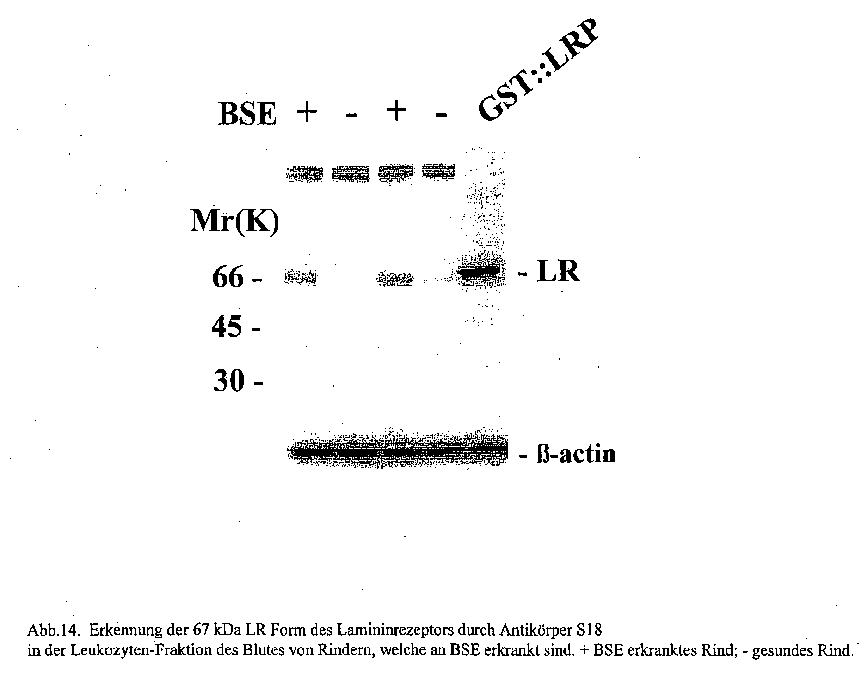

Abb. 14 zeigt den Nachweis eines erhöhten LR Spiegels in der Leukozytenfraktion des Blutes von Rindern, welche an BSE erkrankt sind im Western Blot durch den ScFv S18. Die Blutproben (500 µl) von an BSE erkrankten Rindern sowie gesunden Rindern wurden 1: 1 mit 1 x SSC gemischt, Zentrifugation bei 4000 rpm/10 min. Abnahme des Überstands, Pellet in 1 x SSC resuspendieren, erneut zentrifugieren und Überstand abnehmen. Pellet so lange waschen bis Pellet weiß ist. Das weiße Leukozytenpellet wird in 100 µl TBS resuspendiert. Die Leukozyten werden auf einem 12.5 %igen PA-Gel analysiert. Als Kontrolle wird GST::LRP aus dem Baculovirusexpressionssystem aufgetragen. Die Proteine werden auf PVDF-Membran geblottet und mit dem scFv Antikörper S18 (1:1000; etwa 2 µg/ml) sowie als Kontrolle zur Detektion des ß-Actin-Spiegels mit einem anti-ß-Actin-Antikörper (Chemicon) abgegriffen. Für scFv S18: Sekundärantikörper, anti-c-myc (1:1000), Tertiärantikörper Ziege-anti-Maus-IgG-HRP gekoppelt (1:5000). GST::LRP wurde über den W3 Antikörper, Sek. Antikörper anti-rabbit-IgG-HRP gekoppelt. Die Abbildung zeigt einen erhöhten LR-Spiegel in der Leukozytenfraktion von an BSE leidenden Rindern.

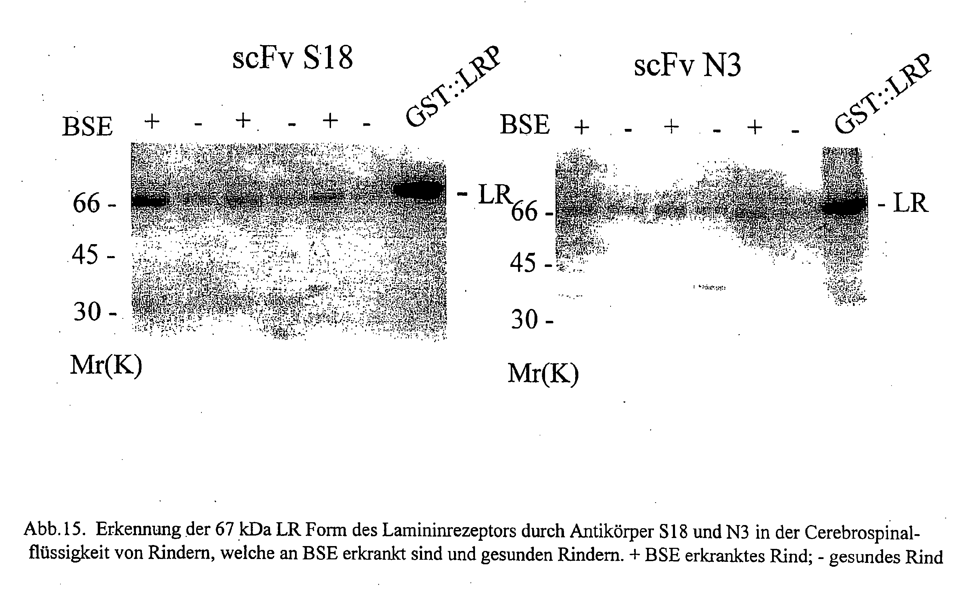

Abb.15 zeigt den Nachweis eines erhöhten LR-Spiegels in der Cerebrosinalflüssigkeit (CSF; Liquor) von an BSE erkrankten Tieren im Vergleich zu gesunden Kontrolltieren. Es wurden gleiche Proteinmengen aufgetragen. Als Positivkontrolle wurde GST::LRP (rec. aus Baculovirussystem) aufgetragen. Gesamtliquor wurde auf einem 12.5 %igem PA-Gel aufgetrennt und auf PVDF Membran geblottet. ScFv S18 und N3 jeweils 1:1000 verdünnt (je ca. 2 µg/ml), Sekundärantikörper Maus-anti-c-myc (1:1000), Tertiäraritikörper Ziege-anti-Maus-IgG-HRP gekoppelt. Nachweis über Chemilumineszenz. Die Abbildung zeigt einen erhöhten LR-Spiegel detektiert durch scFv S18 und N3 in der CSF von an BSE erkrankten Rindern.

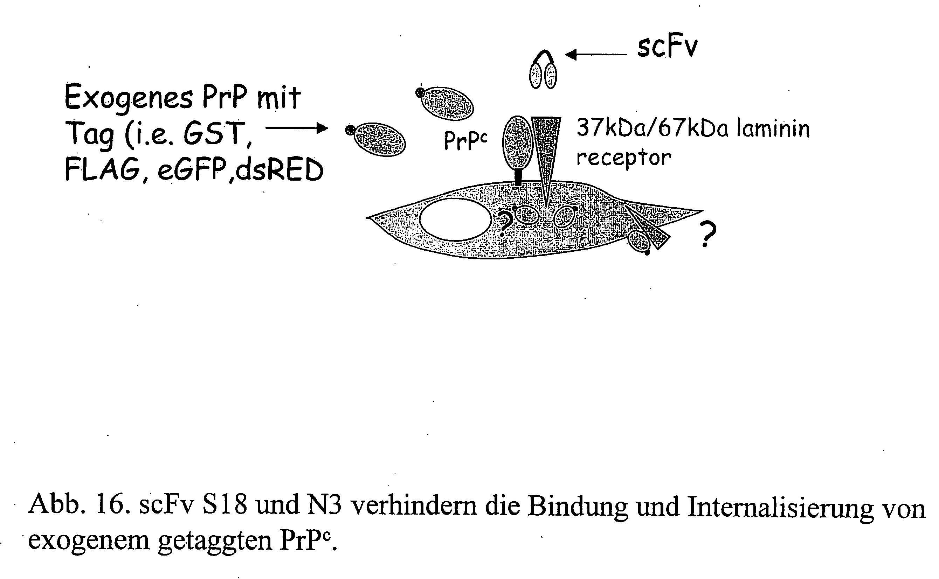

Abb.16 zeigt schematisch, dass scFv S18 und N3 die Bindung und Internalisierung von exogenem getaggten PrP^{c} verhindern können. Die Abb. zeigt einen möglichen Wirkmechanismus der svFv S18 und N3, nämlich der Blockierung der Aufnahme der zellulären Form des Prionproteins.

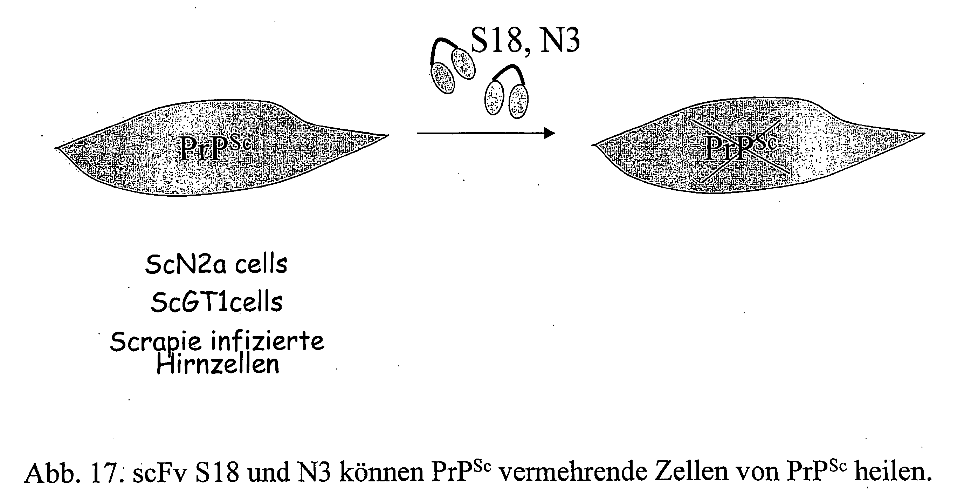

Abb. 17 zeigt schematisch, dass zu mit Scrapie infizierten Zellen gegebene scFv Antikörper S18 und N3 die Zellen von einer Scrapie-Infektion heilen können (Therapeutischer Wirkmechanismus).

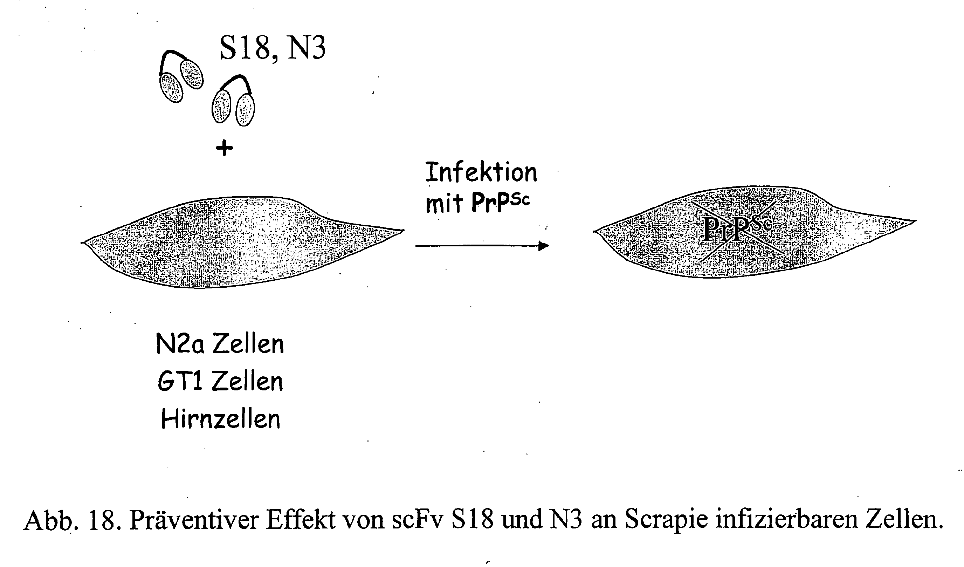

Abb. 18 zeigt schematisch eine prophylaktische Wirkungsweise der scFv S18 und N3. Werden beide Antikörper zu nicht-infizierten Zellen gegeben, verhindern sie, dass die Zellen mit Scrapie infiziert werden können (Prophylaktischer Wirkmechanismus).

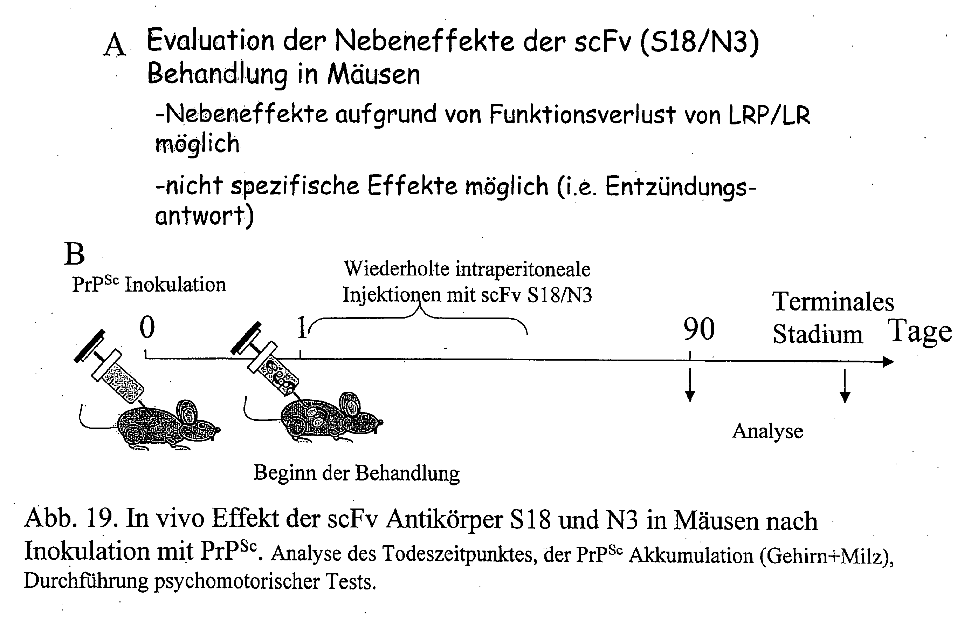

Abb. 19 zeigt schematisch die therapeutische in vivo-Anwendung der scFv Antikörper S18 und N3 im Tier (Passiver Antibody Transfer). (A) Die Antikörper werden zunächst in gesunde Tiere vorzugsweise Mäuse oder Hamster injiziert vorzugsweise intraperitoneal, um mögliche spezifische und nichtspezifische (d.h. Entzündungsantwort) Nebeneffekte der Antikörper zu detektieren. (B) Die scFv Antikörper S18 und N3 werden dann zu verschiedenen Zeitpunkten nach einer PrPSc Inokulation - vorzugsweise ein Tag nach der PrP^{Sc}-Inokulation mit der intraperitonealen Applikation begonnen. Weiterhin werden die Antikörper nach folgendem Protokoll wiederholt appliziert: Eine initiale Dosis von jeweils 200 µg der S18 und N3 Antikörper wird intraperitoneal (i.p.) injiziert in vorzugsweise C57/BL6 Mäuse ein Tag nach intraperitonealer Inokulation mit einem Prion-Stamm vorzugsweise dem BSE Stamm 6PB1. Es folgen i.p. Injektionen zweimal pro Woche mit 100 µg der scFv S18/N3 für weitere vorzugsweise acht Wochen. Ein Teil der Tiere wird 90 Tage p.i. getötet und auf PrP^{Sc} Anwesenheit biochemisch untersucht. Ein anderer Teil der Tiere wird entweder zum terminalen Stadium einer TSE-Erkrankung untersucht oder falls keine Symptome auftraten am Ende der Lebenszeit.

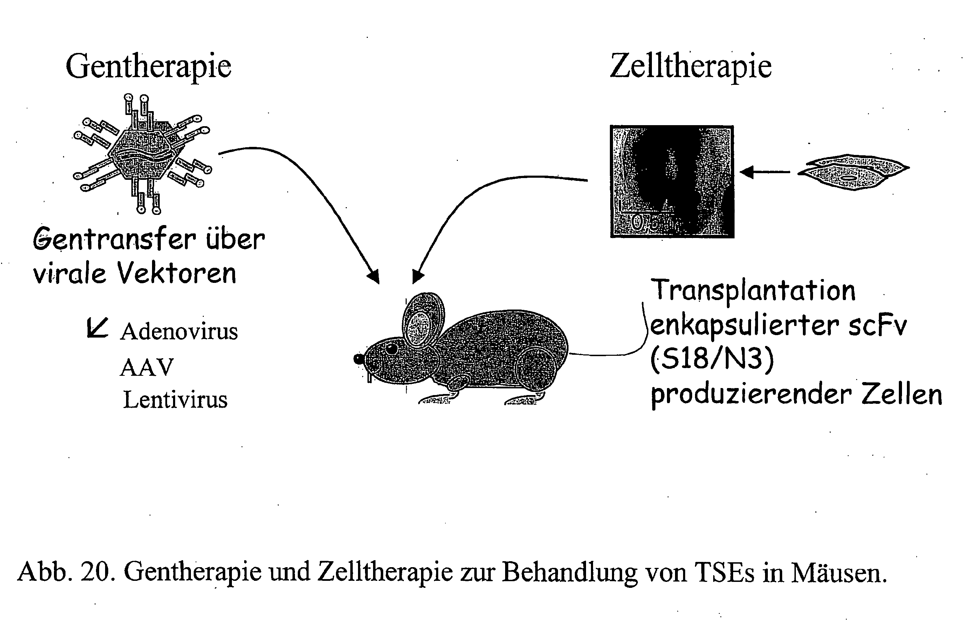

Abb. 20 zeigt den Gentherapie- und Zelltherapie-Ansatz zur Behandlung von Prionerkrankungen mit Hilfe der scFv Antikörper S18 und N3. Der Gentherapeutische Ansatz verwendet virale Vektoren vorzugsweise AAV, um Gene, welche für S18 und N3 kodieren, in Tiere oder Menschen zu transferieren. Der zelltherapeutsiche Ansatz erfolgt durch Transplantation von enkapsulierten Zellen, welche die scFv Antikörper S18 und N3 sezernieren. Beide Ansätze werden in den nachfolgenden Abbildungen näher erläutert.

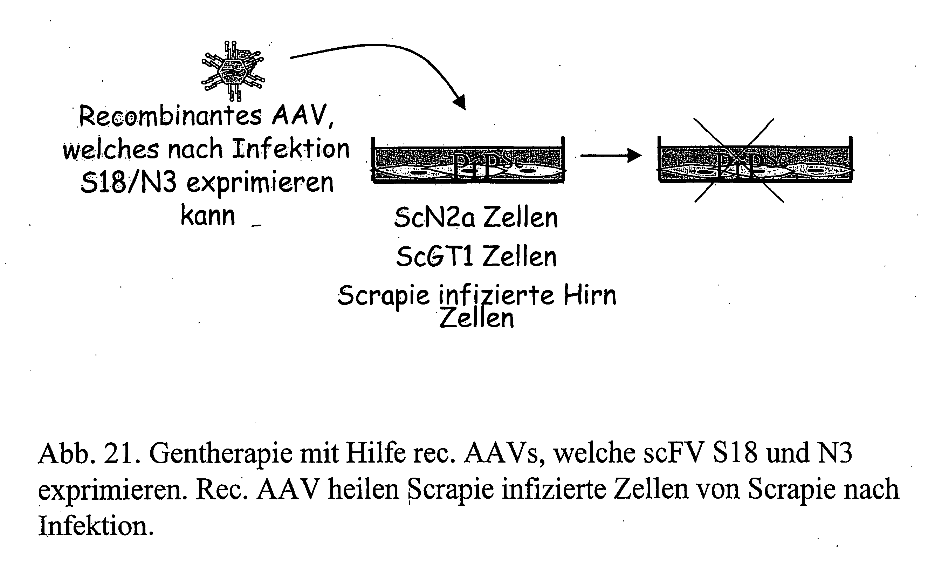

Abb.21 zeigt die Anwendung rekombinanter AAV Viren, welche scFv S 18/N3 exprimieren können, an mit Scrapie infizierten Zellen. Die Infektion von mit Scrapie infizierten neuronalen Zellen mit rekombinanten S18/N3 exprimierenden AAV Viren sollen diese Zellen von Scrapie heilen.

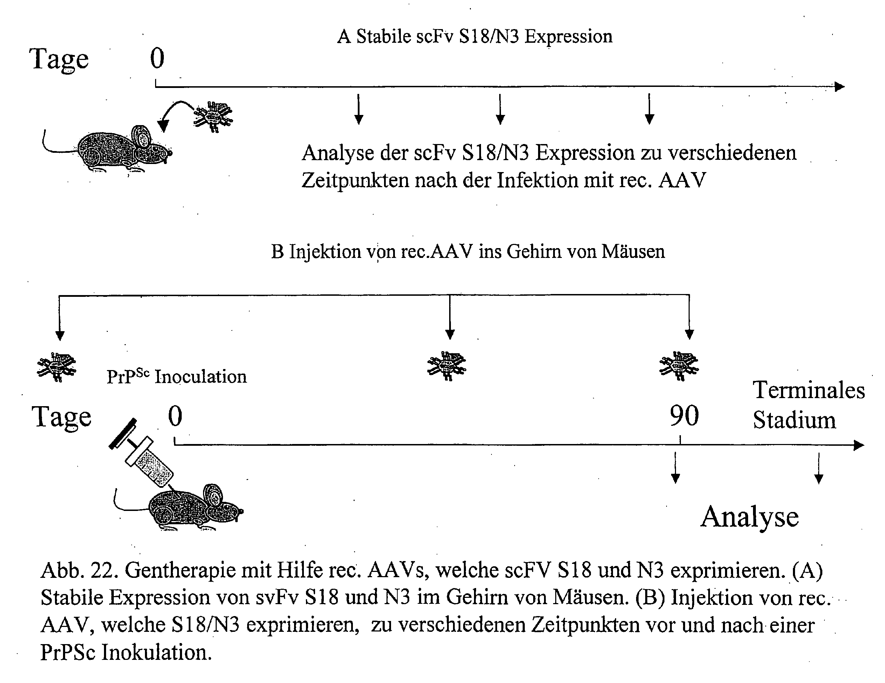

Abb.22 zeigt den gentherapeutischen Ansatz mit rekombinanten AAV Viren, welche S18/N3 exprimieren, in vivo in Säugetieren (einschliesslich dem Menschen) vorzugsweise Mäusen. (A) Nach Injektion der rekombinanten AAV Viren direkt ins Gehirn von gesunden Tieren, wird die Expression der scFV Antikörper S18 und N3 durch Western Blot Analyse der Gehrinfraktionen überprüft. (B) Mäuse werden vor, während oder nach einer PrP^{Sc} Inokulation mit rekombinanten S18/N3 exprimierenden AAV Viren injiziert. Eine Verzögerung des Ausbruchs einer TSE-Erkrankung der Mäuse wird durch psychomotorische Tests und histologische und immunohistochemische Analyse des Gehirns festgestellt.

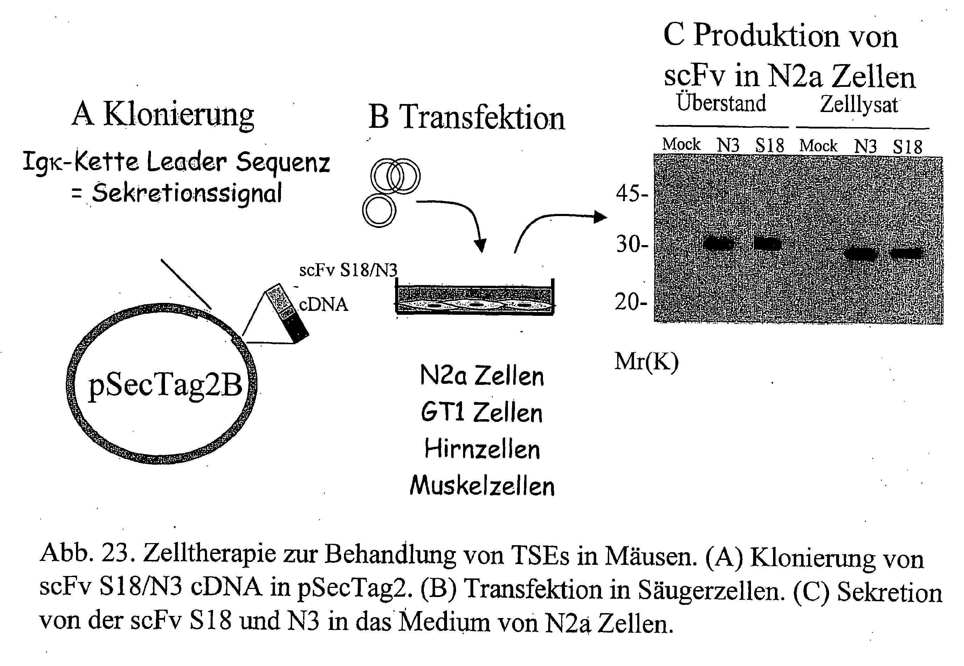

Abb.23 zeigt die Klonierung und Expression/Sekretion von scFv Antikörper S18 und N3 von neuronalen Zellen und schematisch von Muskelzellen. (A) Die cDNA, welche für S18 und N3 kodiert wird über die Restriktionsstellen *Hind*III/*Not*I in den Sekretionsvektor pSecTag2B kloniert. (B) Neuronale Zellen werden mit rec. pSecTag2B, welcher S18 und N3 kodiert, transfiziert. (C) Transfizierte Zellen zeigen 24 Stunden post transfektion (p.t.) die Expression und Sekretion von S18 und N3 durch Western Blotting (1. Antikörper: anti-c-myc; 2. Antikörper: anti-Maus-IgG-HRP konjugiert. Detektion über Chemilumineszenz). Die Abbildung zeigt, dass die scFv S18 und N3 von N2a Zellen sezerniert werden können.

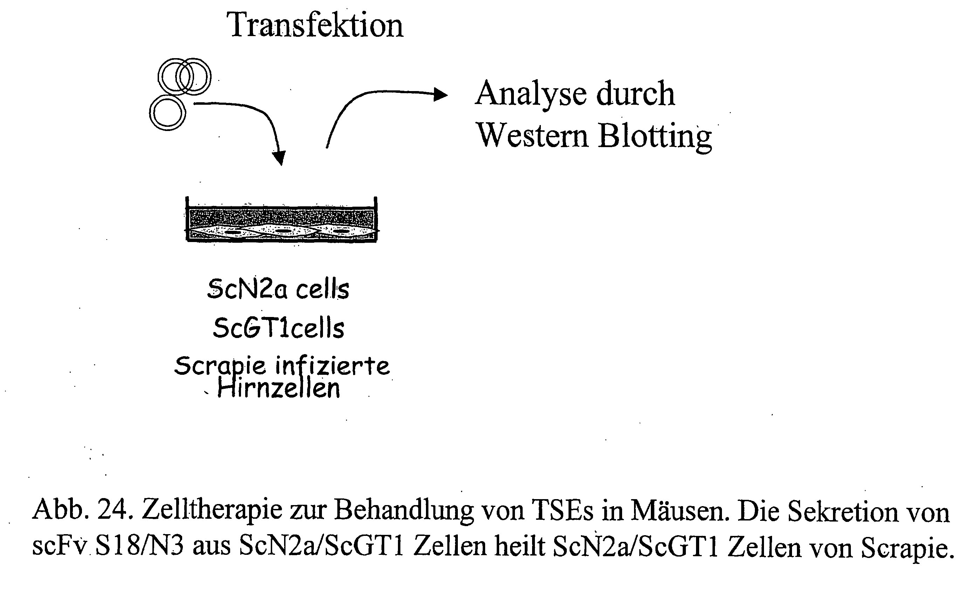

Abb. 24 zeigt die Anwendung S18/N3 sezernierender ScN2a Zellen. Nach Transfektion Scrapie propagierender Zellen sollen diese von PrP^{Sc} geheilt werden.

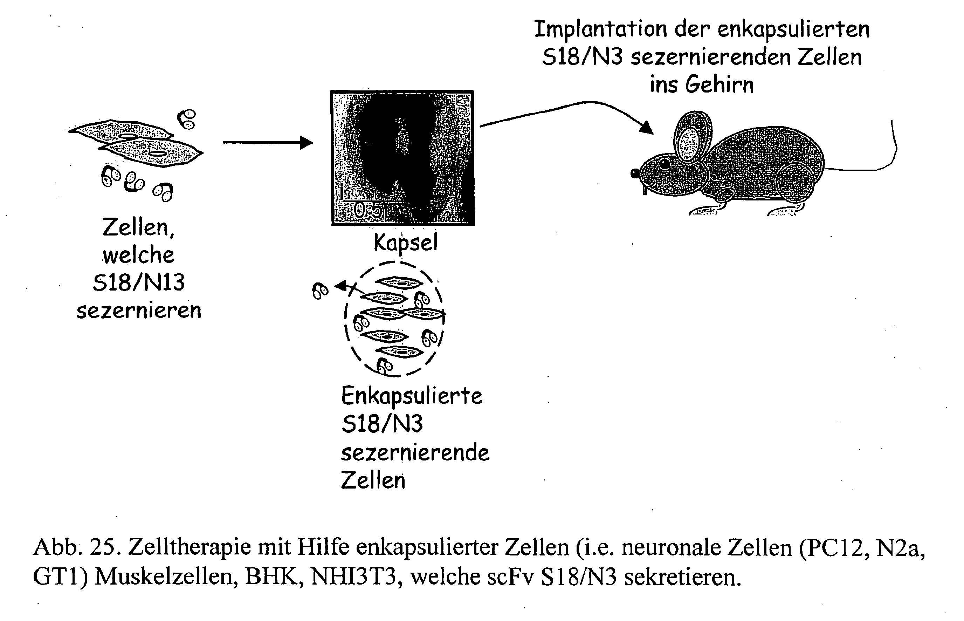

Abb. 25 zeigt schematisch die Zelltherapie durch scFv S18/N3 sezernierende Zellen vorzugsweise Muskelzellen oder neuronale Zellen, BHK Zellen oder NIH3T3 Zellen, welche enkapsuliert sind und in das Gehirn von Säugetieren einschliesslich dem Menschen transplantiert werden. Durch die Sekretion der scFv S 18/N3 soll die Prion-Propagation im Gehirn des Organismus gestoppt werden.

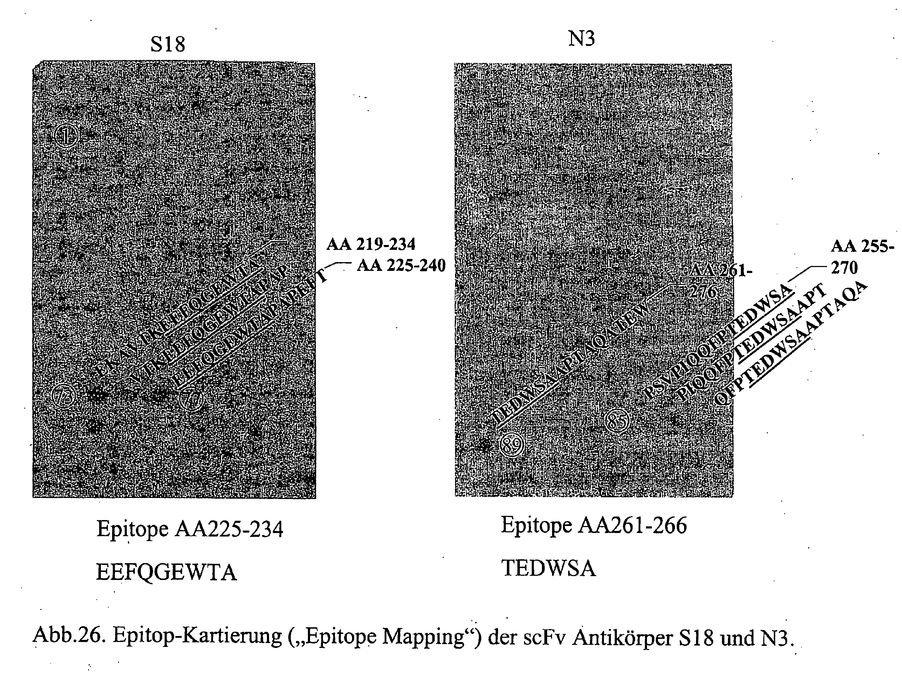

Abb. 26 zeigt die Epitop-kartierung der scFv S 18/N3 Antikörper.

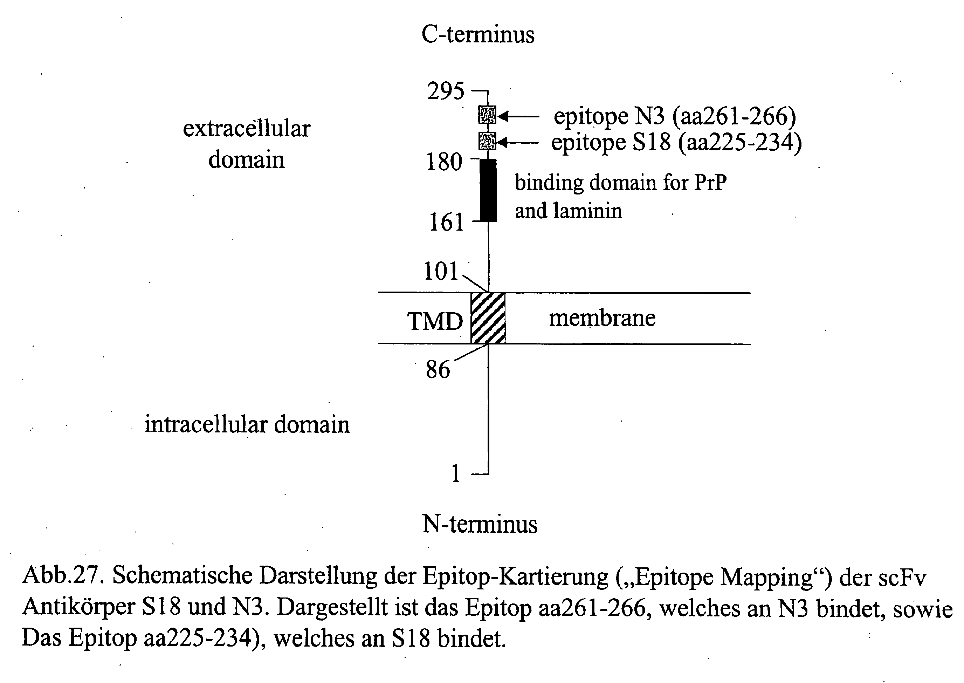

Abb. 27 zeigt eine schematische Darstellung der Epitop-kartierung der scFv S18/N3 Antikörper.

### Verzeichnis der Abkürzungen:

Ab = Antikörper
AAV = Adeno-Associated-Virus

BHK = Baby Hamster Kidney Zellen
BSE = bovine spongiforme Enzephalopathie
BSE+ = Rind, welches an BSE erkrankt ist
BSE- = Rind, welches gesund ist
CDR1/2/3 = complementary determining region 1/2/3
(s/f/nv) CJD = (sporadic/familial/new variant) Creutzfeldt-Jakob-Disease

c-myc = Epitop aus dem korrespondierenden Oncogen
CMV = Cytomegalovirus
CWD = Chronic wasting disease
cy2 = 4'-6-diamidine-2-phenylindole
dsRed = Red Fluorescence protein
eGFP = enhanced Green Fluorescence Protein
ELISA = Enzym Linked Immunoabsorbant Assay
F_{ab} = Antigenbindestelle eines Antikörpers
F_{c} = konstante Region eines Antikörpers
FFI = fatal familial insomnia
FITC = fluorescein isothiocyanat
FLAG = Polypeptid bestehend aus den acht Aminosäuren DYKDDDDK
GSS = Gerstmann-Sträussler-Scheinker Syndrom
GST = Glutathion-S-Transferase
GST::LRP = Fusion aus GST (N-terminal) mit LRP (C-terminal)
Igκ = κ-Kette der Immunglobulin
i.p. = intraperitoneal
Jurkat-Zellen = humane, periphere Blutleukämie-T-Zellen
N1-47 = scFv Antikörper selektiert aus der naiven scFv Bank
His = Histidin
HRP = Horse Raddish Peroxidase
Linker = Aminosäuresequenz, welche zwei Proteindomänen verbindet
LR = 67 kDa Form des Lamininrezeptors (high affinity Lamininrezeptor)
LRP = Lamininrezeptor Precursor
Mr(K) = Molekulargewichtsstandard in kDa
pIII = Phagenhüllprotein
polyHis: Polypeptid bestehend aus sechs Histidin-Resten
PrP^{c} = zelluläre Form des Prionproteins
PrP^{Sc} = Scrapie-Form des Prionproteins
S1-47 = scFv Antikörper selektiert aus der synthetischen scFv Bank
(Sc)GT1 = (scrapie infizierte) hypothalamic neuronal cells
(Sc)N2a = (scarpie infizierte) Neuroblastomzellen
W3 = Antikörper W3 gerichtet gegen LRP/LR, polyklonal
scFᵥ = single chain Antikörper der variablen Region bestehend aus V_{L} und V_{H} verbunden durch einen Linker
scFᵥ N3 = single chain Antikörper N3 selektiert aus der naiven scFᵥ Bank
scFᵥ S 18 = single chain Antikörper S 18 selektiert aus der synthetischen scFᵥ Bank
SFV = Semliki-Förest-Virus
TSE = transmissible spongiforme Enzephalopathie
V_{L} = Leichte Kette der variablen Region eines Antikörpers
V_{H} = schwere Kette der variablen Region eines Antikörpers

Gegenstand eines ersten Aspekts der vorliegenden Erfindung ist ein Single-chain-Antikörpermolekül, welches spezifisch gegen LRP/LR gerichtet ist und das die Aminosäuresequenz SEQ ID Nr.2 umfasst.

Gegenstand eines weiteren Aspekts der vorliegenden Erfindung ist ein Single-chain-Antikörpermolekül, welches spezifisch gegen LRP/LR gerichtet ist und das die Aminosäuresequenz SEQ ID Nr. 4 umfasst.

In einer bevorzugten Ausführungsform handelt es sich bei dem erfindungsgemäßen Antikörpermolekül um ein solches mit der Bezeichnung S 18, das die Aminosäuresequenz SEQ ID Nr. 2 aufweist.

In einer weiteren bevorzugten Ausführungsform handelt es sich bei dem erfindungsgemäßen Antikörpermolekül um ein solches mit der Bezeichnung N3, das die Aminosäuresequenz SEQ ID Nr. 4 aufweist.

Die erfindungsgemäßen Single-chain-Antikörper (scFv) bestehen aus einer Fusion der variablen schweren Ketten (V_{H}) und der variablen leichten Ketten (V_{L}) eines Antikörpermoleküls. Beide Ketten sind über einen Peptidlinker (YOL) verbunden. ScFvs, welche aus den scFv-Bibliotheken stammen, tragen ein C-terminales Histidin-Tag, welches sowohl zur Detektion des scFv als auch zu dessen Reinigung über IMAC verwendet werden kann (Abb. 1 u. Abb. 2).

Antikörper gegen LRP/LR dienen auch als nützliche Werkzeuge zur Diagnostik von Krebserkrankungen und können auch als Therapeutika in Krebserkrankungen eingesetzt werden. Die in der vorliegenden Anmeldung beschriebenen Single-chain-Antikörper, vorzugsweise die Antikörpermoleküle mit der Bezeichnung S 18 und N3, können auch zur Diagnostik und Therapie von Krebserkrankungen eingesetzt werden.

Abb. 10 zeigt die Zelloberflächenerkennung und. intrazelluläre Erkennung von LRP/LR auf Jurkat-Zellen (humane, periphere Blutleukämie-T-Zellen) durch die Single-chain-Antikörper S 18 und N3. Das Beispiel zeigt, dass die erfindungsgemäß bevorzugten Antikörpermoleküle S 18 u. N3 zur Diagnose von Leukämie geeignet sein können. S18 und N3 können sich aus diesem Befund auch für die Diagnose anderer Krebserkrankungen eignen.

Solche Antikörper sind sowohl für die Diagnose wie auch zur Therapie von übertragbaren spongiformen Encephalopathien (transmissible spongiform encephalopathies, TSE) äußerst geeignet. Deshalb eignen sich auch die hier zum erstenmal beschriebenen Single-chain-Antikörper zur Diagnostik von Prionerkrankungen.

Die erfindungsgemäß bevorzugten Antikörpermoleküle mit der Bezeichnung S 18 bzw. N3 wurden aus komplexen synthetischen und naiven Antikörperbanken mit Hilfe der Phagen-Display-Technologie unter Verwendung des in der WO98/53838 beschriebenen GST::LRP Fusionsproteins als Selektionsantigen gewonnen.

Die zur Selektion der scFv-Antikörper verwendeten komplexen scFv-Banken enthielten zusammen etwa 3x10⁹ individuelle Klone (die naïve Bank enthielt etwa 2 x 10⁹ Klone, die synthetische Bank etwa 1 x 10⁹ Klone). Die naïve IgM Bank wurde durch die Kombination der kodierenden Regionen für die variablen schweren und leichten Ketten nach PCR Amplifikation der respektiven cDNA aus Milz oder PBLs (Periphere Blutlymphozyten) generiert. Zur Herstellung der synthetischen Bibliothek wurden humane scFv-Frameworks ausgewählt, welche sich durch gute Faltungs- und Expressionseigenschaften auszeichnen. Die CDR3-Sequenzen der V_{H}-Kette wurden randomisiert.

Die Affmitätsselektion wurde mit jeder der beiden Banken an einem im Baculovirus-System exprimierten GST::LRP Fusionsprotein durchgeführt. Die Herstellung des GST::LRP Fusionsproteins ist in der WO 98/53838-beschrieben.

Abb. 2 fasst schematisch die Generierung der 2 x 10⁹ verschiedenen Klone für die naïve Bank zusammen. Abb. 3 fasst die Generierung der synthetischen scFv Antikörperbank zusammen, welche eine Komplexität von 1 x 10⁹ Klone aufweist.

Abb.4 fasst schematisch die Selektion von scFv Antikörpern zur spezifischen Bindung an GST::LRP zusammen.

Nach der dritten Selektionsrunde, wurden rohe periplasmatische Extrakte von 48 individuellen Klonen von jeweils jeder Bank im ELISA bezüglich der Erkennung des rekombinanten Fusionsproteins GST::LRP getestet (Abb.5 und 6). 66% der individuellen Klone im Falle der naiven Bank und 53% im Falle der synthetischen Bank zeigten ein positives Signal im ELISA (Abb.7).

Eine wiederholte Testung an rekombinantem GST ergab, dass alle Antikörper nicht GST erkannten, obwohl als Antigen das GST::LRP Fusionsprotein verwendet wurde (Abb.7).

Eine Restriktionsanalyse der für die scFv kodierenden DNAs mit *Bst*NI identifizierte einen Klon aus der naiven Bank als hoch angereichert (Abb.7). Die CDR3 Sequenzen der Klone, welche von der synthetischen Bank angereichert wurden, ergaben zwei verschiedene Konsensus-Sequenzen. Alle Klone wurden in einer Western-Blot-Analyse bezüglich der Erkennung des rekombinanten GST::LRP Fusionsproteins getestet (Abb.8). Als Kontrolle wurde GST in jeder Spur zugesetzt, was zeigte, dass keiner der Klone GST erkannte (Abb.8).

Zwei Klone mit der Bezeichnung N3 (aus der naiven Bank) und S18 (aus der synthetischen Bank) wurden aufgrund der stärksten Anreicherung zur weiteren Affinitätsreinigung an einer Cu²⁺-Chelat-Säule ausgewählt.

Der Single-chain-Antikörper mit der Bezeichnung S 18 wird auf cDNA Ebene von der DNA-Sequenz SEQ ID Nr. 1 kodiert.

Die DNA ist im Plasmid pEX/HAM/LRP-S18 enthalten. Das Plasmid wurde bei der DSMZ, Mascheroder Weg 1b, D-38124 Braunschweig unter der Nummer DSM 15962 am 02.10.2003 hinterlegt. Nach Transformation in E.coli XL1-Blue ist die Produktion des scFv Antikörpers S 18 möglich.

Der Single-chain-Antikörper mit der Bezeichnung S18 zeigt auf Proteinebene die Sequenz SEQ ID Nr. 2.

Der Single-chain-Antikörper mit der Bezeichnung N3 wird auf cDNA Ebene von der DNA-Sequenz SEQ ID Nr. 3 kodiert.

Die DNA ist im Plasmid pEX/HAM/LRP-N3 enthalten. Das Plasmid wurde bei der DSMZ, Mascheroder Weg 1b, D-38124 Braunschweig unter der Nummer DSM 15961 am 02.10.2003 hinterlegt. Nach Transformation in E.coli XL1-Blue ist die Produktion des scFv Antikörpers N3 möglich.

Der Antikörper mit der Bezeichnung N3 zeigt auf Proteinebene die Sequenz SEQ ID Nr. 4.

In einer weiteren Ausführungsform gemäß der vorliegenden Erfindung sind die erfindungsgemäßen Antikörpermoleküle an einer oder mehreren Positionen weiter modifiziert, um die Stabilität zu erhöhen und/oder um ihre biochemischen und/oder biophysikalischen Eigenschaften zu verändern.

Die beiden Single-chain-Antikörper S18 und N3 wurden in verschiedenen biochemischen und zellbiologischen Systemen bezüglich spezifischer LRP/LR Erkennung getestet.

Murine Neuroblastomzellen (N2a) wurden durch Fluorescence-Activated Cell Scanning (FACS) mit Hilfe der LRP/LR spezifischen Antikörper W3 (polyklonal) aus der WO 98/53838 und der scFv Antikörper S18 und N3 auf Zelloberflächenexpression von LRP/LR getestet. N2a Zellen stellen das ideale Zellsystem für die Propagation von Prionen dar. Abb. 9 zeigt den Nachweis von LRP/LR durch die scFv Antikörper S18 und N3 auf der Oberfläche von N2a Zellen. Dies zeigt, dass beide Single-chain-Antikörper S18 und N3 LRP/LR auf der Oberfläche von N2a Zellen spezifisch erkennen.

Der 37 kDa/67 kDa LRP/LR ist auf Tumorgewebe stark exprimiert. Beispielsweise auf der Oberfläche metastasierenden Tumorzellen (Coggin et al., 1999; Rohrer et al., 2001). Abb. 10 zeigt den Nachweis des 37 kDa/67 kDa LRP/LR in und auf der Oberfläche von Jurkat-Zellen mit Hilfe der scFv Antikörper S 18 und N3. Jurkat Zellen stellen humane, periphere Blutleukämie-T-Zellen dar. Dies demonstriert, dass die hier beschriebenen Single-Chain-Antikörper in der Lage sind, LRP/LR auf Tumorzellen zu erkennen und zeigt, dass die bevorzugten erfindungsgemäßen Antikörper mit der Bezeichnung S18 und N3 für die Diagnostik von Krebs geeignet sind.

Die hier beschriebenen scFv-Antikörper mit der Bezeichnung S18 und N3 sind in der Lage, rekombinanten humanen und murinen 37 kDa/67 kDa LRP/LR in der Fusion mit einem am Carboxyterminus von LRP angehängten FLAG-Tag in mit rerekombinanter Semliki-Forest-Virus-RNA transfizierten Baby Hamster Kidney Zellen (Baby-Hamster-Nierenzellen) durch Western Blotting, Immunofluoreszenz und Fluoreszenz-activated Cell Scanning (FACS) zu erkennen. Dem Fachmann auf dem einschlägigen Fachgebiet sind diese Methoden bekannt. Abb. 11 zeigt die Erkennung des murinen und humanen 37 kDa LRP::FLAGs sowie der 67 kDa Form durch den scFv N3 und die Erkennung der 37 kDa LRP::FLAG Form durch den scFv S18 in BHK Zellen durch Western Blotting. Abb. 12 zeigt eine Oberflächenfärbung humaner und muriner LRP/LR exprimierender BHK-Zellen mit Hilfe der scFv Antikörper S18 und N3. Der in der WO 98/53838 beschriebene polyklonale Antikörper W3 ist dazu ebenfalls in der Lage. Beide scFvs S18 und N3 erkennen ebenfalls beide murine und humane LRP/LR Moleküle an der Oberfläche von transfizierten BHK-Zellen in der FACS-Analyse (Abb. 13). Die gezeigten Beispiele demonstrieren, dass scFv S18 und N3 LRP/LR Moleküle hochspezifisch erkennen. Die Tatsache, dass verschiedene LRP/LR Spezies von N3 und S 18 erkannt werden, ist auf die extrem starke Konservierung des Proteins während der Evolution zurückzuführen (Ardini et al., 1998).

Gegenstand eines weiteren Aspekts der vorliegenden Erfindung sind diagnostische Zusammensetzungen, die ein erfindungsgemäßes Antikörpermolekül in Verbindung mit einem akzeptablen Verdünnungsmittel und/oder Träger umfassen.

Die erfindungsgemäß bevorzugten scFv-Antikörper mit der Bezeichnung S18 und N3 können als diagnostische Werkzeuge zur Erkennung von übertragbaren spongiformen Enzephalopathien eingesetzt werden. Die Antikörpermoleküle mit der Bezeichnung S18 erkennen in der Leukozyten-Fraktion des Blutes und die Antikörpermoleküle mit der Bezeichnung S18 und N3 erkennen in der Cerebrospinalflüssigkeit ("Liquor") von an BSE erkrankten Rindern einen erhöhten Spiegel der 67 kDa Form des Lamininrezeptors (Abb. 14 und 15). Beide scFv-Antikörper dienen als Werkzeuge für einen sog. Surrogatmarker-Test für die Erkennung von BSE.

Im Gegensatz zu dem in der WO 98/53838 beschriebenen LRP/LR Antikörper W3 sind die erfindungsgemäß bevorzugt verwendeten Single-chain-Antikörper S18 und N3 spezifischer für LR und zeichnen sich aufgrund ihres monoklonalen Ursprungs durch eine höhere Spezifität aus. Weiterhin können beide scFv in unbegrenzter Menge in E.coli hergestellt werden, während der polyklonale Antikörper W3 nur in begrenzter Menge zur Verfügung steht.

In ähnlicher Weise können die erfindungsgemäß bevorzugt verwendeten Antikörpermoleküle mit der Bezeichnung S18 und N3 auch zur Diagnose von anderen TSEs als BSE, wie Scrapie beim Schaf, Chronic Wasting Disease (CWD) bei Cerviden, nvCJD, sCJD, fCJD, Kuru, Gerstmann-Sträussler-Scheinker (GSS) Syndrom und Fataler Familiärer Insomnia (FFI) beim Menschen verwendet werden.

Gegenstand eines weiteren Aspekts der vorliegenden Erfindung sind pharmazeutische Zusammensetzungen, die ein erfindungsgemäßes Antikörpermolekül in Verbindung mit einem pharmazeutisch akzeptablen Verdünnungsmittel und/oder Träger umfassen.

Die erfindungsgemäßen pharmazeutischen Zusammensetzung eignen sich zur Therapie von übertragbaren spongiformen Enzephalopathien (TSEs). Unter TSEs werden alle bekannten Formen der TSEs verstanden. Der Vorteil der erfindungsgemäßen Antikörpermoleküle im Vergleich zu dem in der WO 98/53838 beschriebenen polyklonalen W3 Antikörper bzw. anderen monoklonalen murinen Antikörpern gleicher Spezifität, liegt im humanen Ursprung der erfindungsgemäßen Antikörpermoleküle und der damit verbundenen niedrigen Immunogenität. ScFv ohne F_{c}-Teil und humanen Ursprungs sind in anbetracht der Therapie von Patienten, welche an einer TSE erkrankt sind, von großem Vorteil wegen der potentiell geringeren Immunogenität.

Die erfindungsgemäßen Antikörpermoleküle können zur Verhinderung der Bindung und Internalisierung von Prion-Proteinen an seinen 37 kDa/67 kDa Lamininrezeptor eingesetzt werden (siehe Abb. 16).

Ferner können die erfindungsgemäßen Antikörpermoleküle zur Behandlung von mit Scrapie infizierten Zellen, wie ScGT1, ScN2a und anderen mit Scrapie infizierbaren Hirnzellen eigesetzt werden (Abb.17). Weiterhin können die erfindungsgemäßen Antikörpermoleküle auch zur Prävention eingesetzt werden (Abb.18). In dieser Ausführungsform sollen sie den Ausbruch einer Prionerkrankung in Zellkultur verhindern.

In einer weiteren Ausführungsform können die erfindungsgemäßen Antikörpermoleküle in vivo im Tier eingesetzt werden, um Tiere wie Nager (Hamster Mäuse) von einer Prion-Infektion bzw, einer übertragbaren spongiformen Enzephalopathie zu heilen. Zunächst wie in Abb. 19 ausgeführt, werden mögliche Nebeneffekte der erfindungsgemäßen Antikörpermoleküle evaluiert, indem die erfmdungsgemäßen Antikörpermoleküle in gesunde Mäuse oder Hamster injiziert werden. Die Injektion erfolgt subkutan oder direkt ins Gehirn. Dem Fachmann sind die verschiedenen Injektionsmöglichkeiten von Antikörpern in Säuger geläufig. Nebeneffekte (Nebenwirkungen) der Antikörper werden nach bestimmten Zeitpunkten post-injektionell bis zum Lebensende der Maus (ca. 800 Tage) verfolgt. In einer weiteren Ausführungsform wie in Abb. 19 dargestellt, werden die erfindungsgemäßen Antikörper zu bestimmten Zeitpunkten nach einer Inokulation der Nager mit PrP^{Sc} in die Nager injiziert. Eine eventuelle Verzögerung der Ausbruch einer TSE oder eine Verhinderung eines TSE-Ausbruchs wird beobachtet durch die Analyse des Todeszeitpunktes, der PrP^{Sc} Akkumulation (Gehirn + Milz) und durch Durchführung psychomotorischer Tests. Diese Methoden sind dem Fachmann auf dem einschlägigen Fachgebiet bekannt.

Gegenstand eines-weiteren Aspekts der vorliegenden Erfindung ist die Verwendung der erfindungsgemäßen Antikörper im Rahmen einer Gentherapie und Zelltherapie (Abb. 20). Der Gentherapieansatz führt die Gene, welche für die erfindungsgemäßen Antikörpermoleküle kodieren, in den zu therapierenden Organismus ein. Mehrere Strategien sind bisher zum Gentransfer in neuronale Zellen mit viralen Vektoren (Lentiviren, Adenoviren, Adeno-associated Viren (AAV) verfolgt worden. Dabei war das Adeno-associated-Virus (AAV) System das vielversprechendste. AAV ist nicht-pathogen und kann nicht-teilende Zellen wie Neurone infizieren. Der Gentransfer mit AAV ins zentrale Nervensystem (ZNS) ist effizient und geschieht ohne Aktivierung der zellulären oder humoralen Immunantwort. Der Gentransfer mit AAV wurde in verschiedenen Tiersystemen neurologischer Dysfunktionen erreicht, wie Parkinson's disease (Kirik et al., 2002; Mandel et al., 1997), Alzheimer's disease (Klein et al., 2000), demyelining disease (multiple sclerosis) (Guy et al., 1998), und gelang auch zur Behandlung von Hirntumoren (Ma et al., 2002).

In einer weiteren Ausführungsform können die erfindungsgemäßen Antikörpermoleküle im Gehirn durch rekombinante AAV-Viren exprimiert werden. Unter den AAV-Serotypen ist AAV2 der am höchsten adaptierte und transduziert präferentiell neuronale Zellen. Ein AAV-Vektor, präferentiell ein AAV-2 Vektor, welcher für die erfindungsgemäßen Antikörpermoleküle kodiert, wird verwendet, um hoch-titrige Virionen nach der Methode von Grimm zu produzieren (Grimm et al., 1998). 293 Zellen (human embryonic kidney cell line) werden mit dem AAV-Vektor, welcher für ein erfindungsgemäßes Antikörpermolekül kodiert, zusammen mit einem AAV-Helferplasmid (pDG) co-transfiziert, welches die AAV Hüllprotein-Gene und weitere Adeno-associated Virus Gene exprimiert, welche für Helferfunktionen im Verpacken nötig sind. Mit Scrapie infizierte neuronale Zellen (ScGT1, ScN2a und weitere mit Scrapie infizierte Gehirnzellen) werden mit rekombinanten AAV Viren, welche die erfindungsgemäßen Antikörpermoleküle exprimieren, infiziert, um zu zeigen, dass die Viren die Zellen von Scrapie heilen können (Abb. 21). Die rekombinanten AAV-Viren werden dann in das Gehirn von Mäusen vorzugsweise C57B16, injiziert (Abb.22). Die Expression der erfindungsgemäßen Antikörpermoleküle wird zu verschiedenen Zeitpunkten nach der Infektion über Western Blot Analyse der Gehirnfraktion überprüft (Abb.22A). Rekombinante AAV-Viren werden zu verschiedenen Zeitpunkten vor und nach der Inokulation mit PrP^{Sc} injiziert (Abb.22B). Eine Verzögerung des Ausbruchs einer TSE-Erkrankung der Mäuse wird durch psychomotorische Tests und histologische und immunohistochemische Analyse des Gehirns festgestellt.

Gegenstand einer weiteren Ausführungsform der vorliegenden Erfindung sind pharmazeutische Zusammensetzungen, die die erfindungsgemäßen Antikörpermoleküle produzierende Zellen umfassen, wobei die pharmazeutischen Zusammensetzungen geeignet sind, direkt ins Gehirn von Säugetieren eingebracht zu werden. Beispielsweise kann es sich bei diesen pharmazeutischen Zusammensetzungen um Kapseln handeln, die die erfindungsgemäßen Antikörper produzierende Zellen enthalten. Diese Zusammensetzungen dienen dazu, Säugetiere einschliesslich dem Menschen zu therapieren, welche an einer TSE erkrankt sind. Diese Strategie impliziert, genetisch veränderte Zellen zu benutzen, welche in der Lage sind ein Protein, im vorliegenden Falle die erfindungsgemäßen Antikörpermoleküle zu sezernieren (Abb.20). Die Zellen werden in ein immunoprotektives Polymer z.B. Zellulosesulfat eingekapselt, dessen Poren es erlauben grösse Moleküle, wie im vorliegenden Fall Antikörper, freizusetzen. Dabei bleiben die Zellen lebend über einen langen Zeitraum. Zur Übersicht über diese Technik siehe auch den Artikel von Pelegrin et al., 1998. Diese Strategie wurde bereits erfolgreich zur Behandlung muriner viraler Erkrankungen (Pelegrin et al., 2000) und menschlicher Erkrankungen im Tiermodell wie Parkinson's disease in Primaten (Date et al., 2000) und Huntington's disease in Ratten (Emerich et al., 1996) eingesetzt.

Dieses Verfahren erfordert folgende Schritte: Neuroblastomzellen oder andere neuronale Zellen (PC 12) werden mit einem Expressionsvektor wie z.B. pSecTag2 (Abb. 23) transient oder stabil transfiziert. Zur Sekretion wird die Ig-κ-Kette Leadersequenz verwendet. Für die Expression in neuronalen Zellen verwendet man Promotoren, wie CMV (Cytomegalovirus). Die Sekretion der erfmdungsgemäßen Antikörper von N2a Zellen wurde bereits nachgewiesen (Abb. 23), was demonstriert, dass das Verfahren mit den erfindungsgemäßen Antikörpern funktioniert. Mit Scrapie infizierte neuronale Zellen, werden weiterhin mit den erfindungsgemäßen Sekretionsvektoren transfiziert. Durch die Sekretion beider Antikörper von diesen Zellen, können die Zellen von Scrapie geheilt werden (Abb.24).

Zur Transplantation enkapsulierter Zellen in das Gehirn von Säugetieren einschließlich Mensch zur Therapie von TSEs werden Muskelzellen (vorzugsweise C2.7 Zellen) verwendet, da diese in der Lage sind über einen langen Zeitraum Antikörper zu sezernieren, wenn sie in Mäuse transplantiert sind. Myoblasten oder differenzierte Muskelzellen (vorzugsweise C2.7 Zellen) werden mit einem Expressionsvektor, welcher die erfindungsgemäßen Antikörper unter Kontrolle eines Muskelzell-spezifischen Promotors exprimiert, stabil transfiziert (Abb. 23). Alternativ können auch neuronale Zellen (PC12 Zellen) oder Baby-Hamster Nieren (BHK)-Zellen oder NIH3T3-Zellen, welche die erfindungsgemäßen Antikörper sezernieren können, zur weiteren Enkapsulierung und Transplantation verwendet werden. Die die erfindungsgemäßen Antikörpermoleküle exprimierenden Zellen werden in dem Fachmann auf dem einschlägigen Fachgebiet bekannter Weise enkapsuliert. Das Verfahren ist in der Übersicht bei Pelegrin et al., 1998 beschrieben (Pelegrin et al., 1998). Als Material kann hier z.B. Cellulosesulfat verwendet werden (Pelegrin et al., 1998).

Die enkapsulierten Zellen werden in dem Fachmann auf diesem Fachgebiet bekannter Weise in Gehirne von Mäusen transplantiert. Wie im Falle des oben beschriebenen Gentherapie Ansatzes mit AAV-Viren, wird zunächst die Expression der Single-chain-Antikörper getestet. Um den therapeutischen Effekt der erfindungsgemäßen Antikörper gegenüber einer TSE Erkrankung zu überprüfen, werden die mit die erfindungsgemäßen Antikörpermoleküle exprimierenden Zellen transplantierten Versuchstiere vorzugsweise Mäuse oder Hamster mit PrPSc inokuliert. Eine Verzögerung des Ausbruchs einer TSE-Erkrankung der Mäuse wird durch Psychomotorische Tests und histologische und immunohistochemische Analyse des Gehirns festgestellt.

Die vorliegende Erfindung wird im folgenden unter Bezug auf die Zeichnungen anhand von nicht beschränkend wirkenden Beispielen näher erläutert.

### Beispiele

### Beispiel 1

### Selektion der scFv S18 und N3 aus synthetischen und naiven scFv Banken (Biopanning)

Als Antigen zur Selektion von single chain scFv Fragmenten wurde das in der WO98/53838 beschriebene GST::LRP Fusionsprotein verwendet.

Zur Selektion der scFv-Antikörper wurden zwei komplexe scFv-Banken verwendet, welche etwa 3x10⁹ individuelle Klone enthielten (die naïve Bank enthielt etwa 2 x 10⁹ Klone, die synthetische Bank etwa 1 x 10⁹ Klone). Die naive IgM Bank wurde durch die Kombination der kodierenden Regionen für die variablen schweren und leichten Ketten nach PCR Amplifikation der respektiven cDNA aus Milz oder PBLs (Periphere Blutlymphozyten) generiert. Zur Herstellung der synthetischen Bibliothek wurden humane scFv-Frameworks ausgewählt, welche sich durch gute Faltungs- und Expressionseigenschaften auszeichnen. Die CDR3-Sequenzen der V_{H} -Kette wurden randomisiert.

Die Selektion wurde mit jeder der beiden Banken an einem im Baculovirus System exprimierten GST::LRP Fusionsproteins durchgeführt. Die Herstellung des GST::LRP Fusionsproteins ist in der WO 98/53838 beschrieben.

Abb. 2 fasst schematisch die Generierung der 2 x 10⁹ verschiedenen Klone für die naïve Bank zusammen. Abb. 3 fasst die Generierung der synthetischen scFv Antikörperbank zusammen, welche eine Komplexität von 1 x 10⁹ Klone zeigt.

Abb.4 fasst schematisch die Selektion von scFv Antikörpern zur spezifischen Bindung an GST::LRP zusammen.

Nach der dritten Selektionsrunde, wurden periplasmatische Rohextrakte von 48 individuellen Klonen von jeweils jeder Bank im ELISA bezüglich der Erkennung des rekombinanten Fusionsproteins GST::LRP getestet (Abb.5 und 6). 66% der individuellen Klone im Falle der naiven Bank und 53% im Falle der synthetischen Bank zeigten ein positives Signal im ELISA (Abb.7).

Eine wiederholte Testung an rekombinantem GST ergab, dass alle Antikörper nicht GST erkannten, obwohl als Antigen das GST::LRP Fusionsprotein verwendet wurde (Abb.7).

Eine Restriktionsanalyse der für die scFv kodierenden DNAs mit *Bst*NI zeigte einen Klon aus der naiven Bank als hoch-angereichert (Abb.7). Die V_{H}-CDR3 Sequenzen der aus der synthetischen Bank isolierten Klone zeigen zwei verschiedene Konsensus Sequenzen. Alle Klone wurden in einer Western Blot Analyse bezüglich der Erkennung des rek. GST::LRP Fusionsproteins getestet (Abb.8). Als Kontrolle wurde GST eingesetzt, was zeigte, dass keiner der Klone GST erkannte (Abb.8).

Zwei Klone mit der Bezeichnung N3 (aus der naiven Bank) und S 18 (aus der synthetischen Bank) wurden aufgrund der stärksten Anreicherung zur weiteren Affinitätsreinigung an einer Cu²⁺ Chelat-Säule ausgewählt.

Der Single-chain-Antikörper S 18 wird auf cDNA Ebene von der DNA Sequenz SEQ ID Nr. 1 kodiert.

Die DNA ist im Plasmid pEx/HAM/LRP-S18 enthalten. Das Plasmid wurde bei der DSMZ, Mascheroder Weg 1b, D-38124 Braunschweig unter der Nummer DSM 15962 am 02.10.2003 hinterlegt. Nach Transformation in E.coli XL1-Blue ist die Produktion des scFv Antikörpers S 18 möglich.

Der S 18 Antikörper zeigt auf Proteinebene die Sequenz SEQ ID Nr. 2.

Der Single-chain-Antikörper N3 wird auf cDNA Ebene von der DNA Sequenz SEQ ID Nr. 3 kodiert.

Die DNA ist im Plasmid pEX/HAM/LRP-N3 enthalten. Das Plasmid wurde bei der DSMZ, Mascheroder Weg 1b, D-38124 Braunschweig unter der Nummer DSM 15961 am 02.10.2003 hinterlegt. Nach Transformation in E.coli XL1-Blue ist die Produktion des scFv Antikörpers N3 möglich.

Der N3 Antikörper zeigt auf Proteinebene die Sequenz SEQ ID Nr. 4.

Die scFv Antikörper S18 und N3 können durch Transformation der Plasmide pEX/HAM/LRP-S18 und pEX/HAM/LRP-N3 in E.coli Stämmen wie XL1-Blue in großen Mengen dem Fachmann auf dem einschlägigen Fachgebiet an sich bekannter Weise exprimiert werden. Die exprimierten Proteine S18 und N3 können durch IMAC (Immobilized Metal Affinity Chromatography) dem Fachmann auf dem einschlägigen Fachgebiet an sich bekannter Weise gereinigt werden.

Alternativ können die scFv Antikörper S18 und N3 in dem E.coli Stamm RV308 exprimiert werden. Dazu wird die für S18 und N3 kodierende cDNA aus den Plasmiden pEX/HAM/LRP-S18 und pEX/HAM/LRP-N3 in den Vektor pSKK-2 über *Nco*I/*Not*I kloniert, was zu den Expressionsplasmiden pSKK-S-2-18 und pSKK-2-N3 führte.

Die Expression der scFv S18 und N3 aus pSKK-2 birgt den Vorteil, daß kein PIII-Fusionsprotein mehr produziert wird. Darüberhinaus wird von pSKK-2 das Chaperon SKP exprimiert, welches zur Verbesserung der Proteinfaltung beiträgt. Ferner wird ein zusätzliches c-myc Tag am C-Terminus eingeführt, welche eine alternative Detektion über einen anti-c-myc Antikörper zusätzlich ermöglicht. Der Vorteil des RV308 gegenüber XL-1 Blue ist sein schnelleres Wachstum.

Zur Expression der scFv Antikörper S18 und N3 werden Übernachtkulturen von E. coli RV308 mit pSKK-S18/N3 transformiert (2YT Medium mit 100µg/ml Ampicilin und 50mM Glucose). Es folgt die Verdünnung der Kultur 1:10 in 2YT Medium mit 100µg/ml Ampicilin und 50mM Glucose, die Kultivierung bis die OD₆₀₀ von 0,6-0,8 erreicht ist ( 26°C, 160rpm, ca. 2h). Die Kultur wird bei 7500 rpm, 20°C, 20min abzentrifugiert. Das Pellet wird in 1Vol YTBS mit 100µg/ml Ampicilin, 0,2mM IPTG resuspendiert. Es folgt Inkubation über Nacht bei 21 °C, 160rpm. Dann folgt Zentrifugation bei 9000rpm, 4°C, 20min. Das Pellet wird in 1/20 Vol mit kaltem TES Puffer resuspendiert. Es folgt Inkubation 1 h auf Eis bei gelegentlichem Schütteln. Zentrifugation bei 9000rpm, 4°C, 1h. Der Überstand wird über Nacht gegen PBS dialysiert (4°C). Die dialysierte Antikörperlösung wird bei 9000rpm, 4°C, 1h zentrifugiert. Die Reinigung der Antikörper S18 und N3 erfolgt über eine IMAC (Immobilized Metal Affinity Chromatography). Die Antikörper S18 und N3 werden an "Chelating Sepharose" Beads über Nacht bei 4°C gebunden. Die Beads werden mit Dialysepuffer bei 4°C gewaschen, es folgt das Waschen der Beads mit Waschpuffer bei 4°C. Die sc Fvs S18 und N3 werden mit Elutionspuffer (Imidazol) bei 4°C eluiert. Die Analyse der scFv Antikörper S18 und N3 auf einem 12 %igem SDS PA-Gel gefärbt mit Coomassie Blue ergibt Banden in Höhe von etwa 30 kDa. Nach dem Blotten der Banden auf PVDF-Membran, konnten beide scFv Antikörper über anti-c-myc und anti-His Antikörper nachgewiesen werden.

Die so hergestellten scFv Antikörper S 18 und N3 können für weitere Anwendungen in den folgenden Beispielen eingesetzt werden.

### Beispiel 2

Charakterisierung der scFv Antikörper S18 und N3 an kultivierten Zellen durch FACS, IF und Western Blotting - Nachweis eines erhöhten LRP/LR Spiegels an Tumorzellen (Jurkat Zellen)

Murine Neuroblastomzellen (N2a) wurden durch Fluorescence-Activated Cell Scanning (FACS) mit Hilfe der LRP/LR spezifischen Antikörper W3 (polyklonal) aus der WO 98/53838 und der scFv Antikörper S18 und N3 auf Zelloberflächenexpression von LRP/LR getestet (Abb.9). N2a Zellen stellen das ideale Zellsystem für die Propagation von Prionen dar. Abb. 9 zeigt den Nachweis von LRP/LR durch die scFv Antikörper S18 und N3 auf der Oberfläche von N2a Zellen. Dies zeigt, dass beide Single-chain-Antikörper S18 und N3 LRP/LR auf der Oberfläche von N2a Zellen spezifisch erkennen. Technische Details sind der Legende der Abb. 9 zu entnehmen.

Der 37 kDa/67 kDa LRP/LR ist auf Tumorgewebe stark exprimiert. Beispielsweise auf der Oberfläche metastasierenden Tumorzellen (Coggin et al., 1999; Rohrer et al., 2001). Abb. 10 zeigt den Nachweis des 37 kDa/67 kDa LRP/LR in und auf der Oberfläche von Jurkat-Zellen mit Hilfe der scFv Antikörper S18 und N3. Jurkat-Zellen stellen humane, periphere Blutleukämie-T-Zellen dar. Dies demonstriert, dass die hier beschriebenen Single-chain-Antikörper in der Lage sind LRP/LR auf Tumorzellen zu erkennen und zeigt, dass die Antikörper S18 und N3 für die Diagnostik von Krebs geeignet sind. Technische Details sind der Legende der Abb. 10 zu entnehmen.

Die hier beschriebenen scFv Antikörper S18 und N3 sind in der Lage rekombinanten humanen und murinen 37 kDa/67 kDa LRP/LR in der Fusion mit einem am Carboxyterminus von LRP angehängten FLAG-Tag in mit rec. Semliki-Forest-Virus RNA transfizierten Baby Hamster Kidney Zellen (Baby-Hamster-Nierenzellen) durch Western Blotting, Immunofluoreszenz und Fluoreszenz-activated Cell Scanning (FACS) zu erkennen. Dem Fachmann auf dem einschlägigen Fachgebiet sind diese Methoden bekannt. Abb. 11 zeigt die Erkennung des murinen und humanen 37 kDa LRP::FLAGs sowie der 67 kDa Form durch den scFv N3 und die Erkennung der 37 kDa LRP::FLAG Form durch den scFv S18 in BHK Zellen durch Western Blotting. Abb. 12 zeigt eine Oberflächenfärbung humaner und muriner LRP/LR exprimierender BHK-Zellen mit Hilfe der scFv Antikörper S 18 und N3. Der in der WO 98/53838 beschriebene polyklonale Antikörper W3 ist dazu ebenfalls in der Lage. Beide scFvs S18 und N3 erkennen ebenfalls beide murine und humane LRP/LR Moleküle an der Oberfläche von transfizierten BHK-Zellen in der FACS-Analyse (Abb. 13). Die gezeigten Beispiele demonstrieren, dass scFv S18 und N3 LRP/LR Moleküle hochspezifisch erkennen. Die Tatsache das verschiedene LRP/LR Spezies von N3 und S18 erkannt werden, ist auf die extrem starke Konservierung des Proteins während der Evolution zurückzuführen (Ardini et al., 1998).

Technische Details sind der Legende der Abbildungen 11,12 und 13 zu entnehmen.

### Beispiel 3

### Anwendung von svFv Antikörper S18 und N3 in der BSE Diagnostik

Die scFv Antikörper S 18 und N3 können als diagnostische Werkzeuge zur Erkennung von übertragbaren spongiformen Enzephalopathien einzusetzt werden. Es wird hier demonstriert, dass scFv S18 in der Leukozyten-Fraktion des Blutes und scFv S18 und N3 in der Cerebrospinalflüssigkeit ("Liquor") von an BSE erkrankten Rindern einen erhöhten Spiegel der 67 kDa Form des Lamininrezeptors erkennen (Abb. 14 und 15). Beide scFv Antikörper dienen als Werkzeuge für einen sog. Surrogatmarker-Test für die Erkennung von BSE. Im Gegensatz zu dem in der WO 98/53838 beschriebenen LRP/LR Antikörper W3 sind die Single-chain-Antikörper S18 und N3 spezifischer für LR und zeichen sich aufgrund ihres monoklonalen Ursprungs durch eine höhere Spezifität aus. Weiterhin können beide scFv in unbegrenzter Menge in E.coli hergestellt werden, während der polyklonale Antikörper W3 nur in begrenzter Menge zur Verfügung steht. Ein großer Vorteil gegenüber monoklonales Antikörpern aus Versuchstieren besteht im humanen Ursprung der isolierten scFv-Moleküle.

Technische Details sind der Legende der Abb.14 u. 15 zu entnehmen.

### Beispiel 4

### Anwendung von svFv Antikörper S18 und N3 in der TSE Therapie

Es wird beansprucht die Single-chain-Antikörper scFv S18 und N3 zur Therapie von übertragbaren spongiförmen Enzephalopathien (TSEs) einzusetzten. Unter TSEs werden alle bekannten Formen der TSEs verstanden. Der Vorteil der scFv S18 und N3 im Vergleich zu dem in der WO 98/53838 beschriebenen polyklonalen W3 Antikörper bzw. anderen monoklonalen murinen Antikörpern gleicher Spezifität, liegt im humanen Ursprung der scFvs und der damit verbundenen niedrigen Immunogenität. ScFv ohne F_{c}-Teil und humanen Ursprungs sind in Anbetracht der Therapie von Patienten, welche an einer TSE erkrankt sind, von großem Vorteil wegen der potentiell geringeren Immunogenität. Außerdem können im Gegensatz zu W3 die scFvs in großer Menge hergestellt werden.

scFv S18 und N3 sollen die Bindung und Internalisierung von Prion-Proteinen an deren 37 kDa/67 kDa Lamininrezeptor -wie in Abb. 16 beschrieben - verhindern.

Die scFv S18 und N3 sollen zur Behandlung von Scrapie infizierten Zellenwie ScGT1, ScN2a und anderen Scrapie infizierbaren Hirnzellen eingesetzt werden (Abb. 17). Weiterhin können die scFv S 18 und N3 Antikörper auch zur Prävention eingesetzt werden (Abb.18). In dieser Ausführungsform sollen sie den Ausbruch einer Prionerkrankung in Zellkultur verhindern.

Die scFvs S18 und N3 sollen in vivo im Tier eingesetzt werden, um Tiere wie Nager (Hamster Mäuse) von einer Prion-Infektion bzw, einer übertragbaren spongiformen Enzephalopathie zu heilen. Zunächst, wie in Abb. 19 ausgeführt, werden mögliche Nebeneffekte der ScFv Antikörper S18 und N3 evaluiert, indem die scFv Antikörper S18 und N3 in gesunde Mäusen oder Hamster injiziert werden. Die Injektion erfolgt intraperitoneal. Dem Fachmann sind die verschiedenen Injektionsmöglichkeiten von Antikörpern in Säuger geläufig. Nebeneffekte (Nebenwirkungen) der Antikörper werden nach bestimmten Zeitpunkten post-injektionell bis zum Lebensende der Maus (ca. 800 Tage) verfolgt. In einer weiteren Ausführungsform wie in Abb. 19 dargestellt, werden die ScFv Antikörper zu bestimmten zeitpunkten nach einer intraperitonealen Inokulation der Nager mit PrP^{sc} in die Nager intraperitoneal injiziert. Protokoll: Eine initiale Dosis von jeweils 200 µg der S18 und N3 Antikörper wird intraperitoneal (i.p.) injiziert in vorzugsweise C57/BL6 Mäuse einen Tag nach intraperitonealer Inokulation mit einem Prion-Stamm vorzugsweise dem BSE Stamm 6PB1. Es folgen i.p. Injektionen zweimal pro Woche mit 100 µg der scFv S18/N3 für weitere vorzugsweise acht Wochen. Ein Teil der Tiere wird 90 Tage p.i. getötet und auf PrP^{Sc} Anwesenheit biochemisch untersucht. Ein anderer Teil der Tiere wird entweder zum terminalen Stadium einer TSE-Erkraükung untersucht oder falls keine Symptome auftraten am Ende der Lebenszeit. Eine eventuelle Verzögerung der Ausbruch einer TSE oder eine Verhinderung eines TSE-Ausbruchs wird beobachtet durch die Analyse des Todeszeitpunktes, der PrP^{Sc} Akkumulation (Gehirn+Milz) und durch Durchführung psychomotorischer Tests. Diese Methoden sind dem Fachmann auf dem einschlägigen Fachgebiet bekannt.

scFv Antikörper S18 und N3 sollen über Gentherapie und Zelltherapie in vivo transferiert werden (Abb. 20). Der Gentherapie-Ansatz führt die Gene, welche für die scFvs kodieren, in den zu therapierenden Organismus ein. Mehrere Strategien sind bisher zum Genübertransfer in neuronale Zellen mit viralen Vektoren (Lentiviren, Adenoviren, Adeno-associated Viren (AAV)) verfolgt worden. Dabei war das Adeno-associated-Virus (AAV) System das vielversprechendste. AAV ist nicht-pathogen und kann nicht-teilende Zellen wie Neurone infizieren. Der Gentransfer mit AAV ins zentrale Nervensystem (ZNS) ist effizient und geschieht ohne Aktivierung der zellulären oder humoralen Immunantwort. Der Gentransfer mit AAV wird in verschiedenen Tiersystemen neurologischer Dysfunktionen erreicht, wie Parkinson's disease (Kirik et al., 2002; Mandel et al., 1997), Alzheimer's disease (Klein et al., 2000), demyelining disease (multiple sclerosis) (Guy et al., 1998), und gelang auch zur Behandlung von Hirntumoren (Ma et al., 2002).

Die scFvs S18 und N3 sollen im Gehirn durch rec. AAV-Viren exprimiert werden. Unter den AAV Serotypen ist AAV2 der am höchsten adaptierte und transduziert präferentiell neuronale Zellen. Ein AAV Vektor, präferentiell ein AAV-2 Vektor, welcher die für scFv S 18 oder N3 kodiert, wird verwendet um Hoch-Titrige Virionen anch der Methode von Grimm zu produzieren (Grimm et al., 1998): 293 Zellen (human embryonic kidney cell line) werden mit dem AAV-Vektor, welche S18 und N3 kodiert, zusammen mit einem AAV-Helferplasmid (pDG) co-transfiziert, welche die AAV Hüllprotein-Gene und weitere Adeno-associated Virus Gene exprimiert, welche für Helfer Funktionen im Verpacken nötig sind. Scrapie infizierte Neuronale Zellen (ScGT1, ScN2a und weitere Scrapie infizierte Gehirnzellen) werden mit rekombinanten AAV Viren, welche scFv S18 und N3 exprimieren, infiziert, um zu zeigen, daß die Viren die Zellen von Scrapie heilen können (Abb. 21). Die recombinanten AAV Viren werden dann in das Gehirn von Mäusen vorzugsweise C57B16, injiziert (Abb.22). Die Expression der scFvs S18 und N3 wird zu verschiedenen Zeitpunkten nach der Infektion über Western Blot Analyse der Gehirnfraktion überprüft (Abb.22A). rec. AAV Viren werden zu verschiedenen Zeitpunkten vor und nach der Inokulation mit PrPSc injiziert (Abb.22B). Eine Verzögerung des Ausbruchs einer TSE-Erkrankung der Mäuse wird durch psychomotorische Tests und histologische und immunohistochemische Analyse des Gehirns festgestellt.

scFv Antikörper S18 und N3 sollen direkt ins Gehirn von Säugetieren eingebracht werden indem man Kapseln transplantiert, welche Antikörper produzierende Zellen enthalten. Diese Ausführungsform dient ebenfalls dazu Säugetiere einschliesslich dem Menschen zu therapieren, welche an einer TSE erkrankt sind. Diese Strategie impliziert genetisch veränderte Zellen zu benutzen, welche in der Lage sind ein Protein, im vorliegenden Falle die scFv Antikörper S18 und N3 zu sezernieren (Abb.20). Die Zellen werden in ein inununoprotektives Polymer z.B. Zellulosesulfat enkapsuliert, dessen Poren es erlauben grosse Moleküle, wie im vorliegenden Fall Antikörper, freizusetzen, Dabei bleiben die Zellen lebend über einen langen Zeitraum. Zur Übersicht über diese Technik siehe (Pelegrin et al., 1998). Diese Strategie wurde bereits erfolgreich zur Behandlung muriner viraler Erkrankungen (Pelegrin et al., 2000) und menschlicher Erkrankungen im Tiermodell wie Parkinson's disease in Primaten (Date et al., 2000) und Huntington's disease in Ratten (Emerich et al., 1996) eingesetzt.

Dieses Verfahren erfordert folgende Schritte: Neuroblastomzellen oder andere neuronale Zellen (PC 12) werden mit einem Expressionsvektor wie z.B. pSecTag2 (Abb. 23) transient oder stabil transfziert. Zur Sekretion wird die Ig-κ-Kette Leadersequenz verwendet. Für die Expression in neuronalen Zellen verwendet man Promotoren, wie CMV (Cytomegalovirus). Die Sekretion der Antikörper S18 und N3 von N2a Zellen wurde bereits nachgewiesen (Abb. 23), was demonstriert, dass das Verfahren mit den scFv Antikörpern S18 und N3 funktioniert. Scrapie infizierte neuronale Zellen, werden weiterhin mit den S18/N3 Sekretionsvektoren transfiziert. Durch die Sekretion beider Antikörper von diesen Zellen, können die Zellen von Scrapie geheilt werden (Abb.24).

Zur Transplantation enkapsulierter Zellen in das Gehirn von Säugetieren einschließlich Mensch zur Therapie von TSEs werden Muskelzellen (vorzugsweise C2.7 Zellen) verwendet, da diese in der Lage sind über einen langen Zeitraum Antikörper zu sezernieren, wenn sie in Mäuse transplantiert sind. Myoblasten oder differenzierte Muskelzellen (vorzugsweise C2.7 Zellen) werden mit einem Expressionsvektor, welcher die scFv Antikörper S 18 oder N3 unter Kontrolle eines Muskelzell-spezifischen Promotors exprimiert, stabil transfiziert (Abb. 23). Alternativ können auch neuronale Zellen (PC12 Zellen) oder Baby-Hamster Kidney (BHK) Zellen oder NIH3T3 Zellen, welche die Antikörper S18/N3 sezernieren können, zur weiteren Enkapsulierung und Transplantation verwendet werden. Die scFv S18/N3 exprimierenden Zellen werden dem Fachmann auf dem einschlägigen Fachgebiet bekannter Weise enkapsuliert. Das Verfahren ist in der Übersicht bei Pelegrin et al., 1998 beschrieben (Pelegrin et al., 1998). Als Material kann hier z.B. Cellulose Sulphat verwendet werden (Pelegrin et al., 1998).

Die enkapsulierten Zellen werden in dem Fachmann auf diesem Fachgebiet bekannter Weise in Gehirne von Mäusen transplantiert. Wie im Falle des oben beschriebenen Gentherapieansatzes mit AAV-Viren, wird zunächst die Expression der Single-chain-Antikörper getestet. Um den therapeutischen Effekt der scFv Antikörper gegenüber einer TSE Erkrankung zu überprüfen, werden die mit scFv S18/N3 exprimierenden Zellen transplantierten Versuchstiere vorzugsweise Mäuse oder Hamster mit PrPSc inokuliert. Eine Verzögerung des Ausbruchs einer TSE-Erkrankung der Mäuse wird durch Psychomotorische Tests und histologische und immunohistochemische Analyse des Gehirns festgestellt.

Die Techniken zu den Ausführungen im Beispiel 4 sind dem Fachmann auf dem einschlägigen Fachgebiet geläufig.

### Beispiel 5

### Epitop-Kartierung ("Epitope Mapping") der scFvs S18 und N3

Diese Technik dient dazu die Epitope auf LRP zu identifizieren, welche von den scFvs S18 und N3 erkannt werden. Dazu wurden 92 verschiedene Peptide synthetisiert, welche jeweils eine Länge von 15 Aminosäuren aufwiesen. Der N-Terminus jedes Peptids wurde um 3 Aminosäuren in Bezug auf das vorhergehende Peptid verschoben, so dass jedes Peptid mit dem vorhergehenden Peptid um 12 Aminosäuren überlappte. Die Synthese der Peptide erfolgte an einer Cellulose-Membran.

Wie in Abb. 26 gezeigt, wurden mit dem Antikörper S 18 drei starke Signale detektiert. Diese entsprechen den Peptiden EKAVTKEEFQGEWTA, VTKEEFQGEWTAPAP, und EEFQGEWTAPAPEFT. Das gemeinsame Epitop ist EEFQGEWTA (AA225-234). Dies bedeutet für den Fachmann, dass sich das Epitop auf LRP für den scFv S 18 von Aminosäure 225 bis 243 auf dem Lamininrezeptor erstreckenkönnte. Dies ist schematisch in Abb. 27 illustriert.

Mit dem Antikörper N3 wurden vier Signale visualisiert, wie in Abb. 26 gezeigt:
PSVPIQQFPTEDWSA, PIQQFPTEDWSAAPT, QFPTEDWSAAPTAQA und TEDWSAAPTAQATEW. Hier ist das gemeinsame Epitop TEDWSA (AA261-266). Dies bedeutet für den Fachmann, dass sich das Epitop auf LRP für den scFv N3 von Aminosäure 261 bis 266 auf dem Lamininrezeptor erstreckenkönnte (vgl. Abb. 27).

Die Techniken zu den Ausführungen im Beispiel 5, welche dem Fachmann auf dem einschlägigen Fachgebiet geläufig sind, werden im folgenden detailliert ausgeführt.

Der Antikörper Bindungstest wurde ähnlich durchgeführt wie Western Blots zur Detektion von LRP durch die Antikörper scFv S18 und N3 (Vgl. Abb. 11). Dabei wurde die mit 92 Peptiden gebundene Membran mit den scFv Antikörper S18 und N3 (Verdünnung 1:5000) inkubiert. Um einen Antikörper (S18) zu entfernen (um mit dem zweiten Antikörper (N3) zu detektieren), wurde die Membran 3 mal 20 Minuten mit "Stripping" Puffer (8 M Harnstoff, 0.5% β-Mercaptoethanol) bei 60°C inkubiert.

### Literaturverzeichnis:

Aguzzi, A. and Weissmann, C. (1998) Prion diseases. Haemophilia, 4, 619-627.
Ardini, E., Pesole, G., Tagliabue, E., Magnifico, A., Castronovo, V., Sobel, M.E., Colnaghi, M.I. and Menard, S. (1998) The 67-kDa laminin receptor originated from a ribosomal protein that acquired a dual function during evolution. Mol. Biol. Evol., 15, 1017-1025.
Auth, D. and Brawerman, G. (1992) A 33-kDa polypeptide with homology to the laminin receptor: component of translation machinery. Proc Natl Acad Sci USA, 89, 4368-4372. Beck, K., Hunter, I. and Engel, J. (1990) Structure and function of laminin: anatomy of a multidomain glycoprotein. FASEB J., 4, 148-160.
Buto, S., Tagliabue, E., Ardini, E., Magnifico, A., Ghirelli, C., van den Brule, F., Castronovo, V., Colnaghi, M.I., Sobel, M.E. and Menard, S. (1998) Formation of the 67-kDa laminin receptor by acylation of the precursor. J. Cell. Biochem., 69, 244-251.
Canfield, S.M. and Khakoo, A.Y. (1999) The nonintegrin laminin binding protein (p67 LBP) is expressed on a subset of activated human T lymphocytes and, together with the integrin very late activation antigen-6, mediates avid cellular adherence to laminin. J Immunol, 163, 3430-3440.
Castronovo, V., Claysmith, A.P., Barker, K.T., Cioce, V., Krutzsch, H.C. and Sobel, M.E. (1991) Biosynthesis of the 67 kDa high affinity laminin receptor. Biochem. Biophys. Res. Commun., 177,177-183.
Coggin, J.H., Jr., Barsoum, A.L. and Rohrer, J.W. (1999) 37 kiloDalton oncofetal antigen protein and immature laminin receptor protein are identical, universal T-cell inducing immunogens on primary rodent and human cancers. Anticancer Res, 19, 5535-5542.
Date, I., Shingo, T., Yoshida, H., Fujiwara, K., Kobayashi, K. and Ohmoto, T. (2000) Grafting of encapsulated dopamine-secreting cells in Parkinson's disease: long-term primate study. Cell Transplant, 9, 705-709.
Davis, S.C., Tzagoloff, A. and Ellis, S.R. (1992) Characterization of a yeast mitochondrial ribosomal protein structurally related to the mammalian 68-kDa high affinity laminin receptor. J Biol Chem, 267, 5508-5514.
Douville, P.J. and Carbonetto, S. (1992) Genetic linkage analysis in recombinant inbred mice of P40, a putative clone for the high-affinity laminin receptor. Mamm. Genome, 3, 438-446.
Emerich, D.F., Lindner, M.D., Winn, S.R., Chen, E.Y., Frydel, B.R. and Kordower, J.H. (1996) Implants of encapsulated human CNTF-producing fibroblasts prevent behavioral deficits and striatal degeneration in a rodent model of Huntington's disease. J Neurosci, 16, 5168-5181.
Enright, A.J., Van Dongen, S. and Ouzounis, C.A. (2002) An efficient algorithm for largescale detection of protein families. Nucleic Acids Res, 30, 1575-1584. Fernandez, M.-T., Castronovo, V., Rao, C.N. and Sobel, M.E. (1991) The high affinity murine laminin receptor is a member of a multicopy gene family. Biochem. Biophys. Res. Commun., 175, 84-90.
Garcia-Hernandez, M., Davies, E. and Staswick, P.E. (1994) Arabidopsis p40 homologue. A novel acidic protein associated with the 40 S subunit of ribosomes. J. Biol. Chem., 269, 20744-20749.
Gauczynski, S., Hundt, C., Leucht, C. and Weiss, S. (2001 a) Interaction of prion proteins with cell surface receptors, molecular chaperones and other molecules. Adv. Prot. Chem., 57, 229-272.
Gauczynski, S., Krasemann, S., Bodemer, W. and Weiss, S. (2002) Recombinant human prion protein mutants huPrP D178N/M129 (FFI) and huPrP+90R (fCJD) reveal proteinase K resistance. J Cell Sci, 115, 4025-4036.
Gauczynski, S., Peyrin, J.M., Haik, S., Leucht, C., Hundt, C., Rieger, R., Krasemann, S., Deslys, J.P., Dormont, D., Lasmezas, C.I. and Weiss, S. (2001b) The 37-kDa/67-kDa laminin receptor acts as the cell-surface receptor for the cellular prion protein. EMBO J, 20, 5863-5875.
Grimm, D., Kern, A., Rittner, K. and Kleinschmidt, J.A. (1998) Novel tools for production and purification of recombinant adenoassociated virus vectors. Hum Gene Ther, 9, 2745-2760.
Guy, J., Qi, X. and Hauswirth, W.W. (1998) Adeno-associated viral-mediated catalase expression suppresses optic neuritis in experimental allergic encephalomyelitis. Proc Natl Acad Sci U S A, 95, 13847-13852.
Hinek, A., Wrenn, D.S., Mecham, R.P. and Barondes, S.H. (1988) The elastin receptor: a galactoside binding protein. Science, 239, 1539-1541.
Hundt, C., Peyrin, J.M., Haik, S., Gauczynski S., Leucht, C., Rieger, R., Riley, M.L., Deslys, J.P., Dormont, D., Lasmezas, C.I. and Weiss, S. (2001) Identification of interaction domains of the prion protein with its 37-kDa/67-kDa laminin receptor. EMBO J, 20, 5876-5886. Jackers, P., Clausse, N., Fernandez, M., Berti, A., Princen, F., Wewer, U., Sobel, M.E. and Castronovo, V. (1996a) Seventeen copies of the human 37 kDa laminin receptor precursor/p40 ribosome-associated protein gene are processed pseudogenes arisen from retropositional events. Biochim. Biophys. Acta, 1305, 98-104.
Jackers, P., Minoletti, F., Belotti, D., Clausse, N., Sozzi, G., Sobel, M.E. and Castronovo, V. (1996b) Isolation from a multigene family of the active human gene of the metastasis-associated multifunctional protein 37LRP/p40 at chromosome 3p21.3. Oncogene, 13, 495-503.
Keppel, E. and Schaller, H.C. (1991) A 33 kDa protein with sequence homology to the laminin binding protein' is associated with the cytoskeleton in hydra and in mammalian cells. J. Cell. Science, 100, 789-797.
Kinoshita, K., Kaneda, Y., Sato, M., Saeki, Y., Wataya, K.M. and Hoffmann, A. (1998) LBPp40 binds DNA tightly through associations with histones H2A, H2B, and H4. Biochem Biophys Res Commun, 253, 277-282.
Kirik, D., Georgievska, B., Burger, C., Winkler, C., Muzyczka, N., Mandel, R.J. and Bjorklund, A. (2002) Reversal of motor impairments in parkinsonian rats by continuous intrastriatal delivery of L-dopa using rAAV-mediated gene transfer. Proc Natl Acad Sci U S A, 99, 4708-4713.
Klein, R.L., Hirko, A.C., Meyers, C.A., Grimes, J.R., Muzyczka, N. and Meyer, E.M. (2000) NGF gene transfer to intrinsic basal forebrain neurons increases cholinergic cell size and protects from age-related, spatial memory deficits in middle-aged rats. Brain Res, 875, 144-151.
Landowski, T.H., Dratz, E.A. and Starkey, J.R. (1995) Studies of the structure of the metastasis-associated 67 kDa laminin binding protein: fatty acid acylation and evidence supporting dimerization of the 32 kDa gene product to form the mature protein. Biochemistry, 34, 11276-11287.
Lasmezas, C.I. and Weiss, S. (2000) Molecular Biology of Prion Diseases. In Cary, J.W., Linz, J.E. and Bhatnagar, D. (eds.), Microbial Foodborne Diseases. Mechanisms of Pathogenicity and Toxin Synthesis. Technomic Publishing CO., INC, Lancaster (USA), pp. 495-537.
Lesot, H., Kühl, U. and von der Mark, K.-(1983) Isolation of a laminin binding protein from muscle cell membranes. EMBO J., 2, 861-865.
Leucht, C., Simoneau, S., Rey, C., Vana, K., Rieger, R., Lasmezas, C.I. and Weiss, S. (2003) The 37 kDa/67 kDa laminin receptor is required for PrP(Sc) propagation in scrapie-infected neuronal cells. EMBO Rep, 4, 290-295.
Leucht, C. and Weiss, S. (2002) Der Prion Protein Rezeptor. Nova Acta Leopoldina, 87, 39-54.
Lopez-Ribot, J.L., Casanova, M., Monteagudo, C., Sepulveda, P. and Martinez, J.P. (1994) Evidence for the presence of a high-affinity laminin receptor-like molecule on the surface of Candida albicans yeast cells. Infect Immun, 62, 742-746.
Ludwig, G.V., Kondig, J.P. and Smith, J.F. (1996) A putative receptor for Venezuelan equine encephalitis virus from mosquito cells. J Virol, 70, 5592-5599.
Ma, H.I., Lin, S.Z., Chiang, Y.H., Li, J., Chen, S.L., Tsao, Y.P. and Xiao, X. (2002) Intratumoral gene therapy of malignant brain tumor in a rat model with angiostatin delivered by adeno-associated viral (AAV) vector. Gene Ther, 9, 2-11.
Malinoff, H.L. and Wicha, M.S. (1983) Isolation of a cell surface receptor for laminin from murine fibrosarcoma cells. J. Cell. Biol., 96, 1475-1479.
Mandel, R.J., Spratt, S.K., Snyder, R.O. and Leff, S.E. (1997) Midbrain injection of recombinant adeno-associated virus encoding rat glial cell line-derived neurotrophic factor protects nigral neurons in a progressive 6-hydroxydopamine-induced degeneration model of Parkinson's disease in rats. Proc Natl Acad Sci USA, 94, 14083-14088.Mecham, R.P. (1991) Receptors for laminin on mammalian cells. FASEB J., 5, 2538-2546.
Melnick, M.B., Noll, E. and Perrimon, N. (1993) The Drosophila stubarista phenotype is associated with a dosage effect of the putative ribosome-associated protein D-p40 on spineless. Genetics, 135, 553-564.
Ouzonis, C., Kyrpides, N. and Sander, C. (1995) Novel protein families in archaean genomes. Nucleic Acids Res, 23, 565-570.
Pelegrin, M., Marin, M., Noel, D., Del Rio, M., Saller, R., Stange, J., Mitzner, S., Gunzburg, W.H. and Piechaczyk, M. (1998) Systemic long-term delivery of antibodies in immunocompetent animals using cellulose sulphate capsules containing antibody-producing cells. Gene Ther, 5, 828-834.
Pelegrin, M., Marin, M., Oates, A., Noel, D., Saller, R., Salmons, B. and Piechaczyk, M. (2000) Immunotherapy of a viral disease by in vivo production of therapeutic monoclonal antibodies. Hum Gene Ther, 11, 1407-1415.
Prusiner, S.B., Scott, M.R., DeArmond, S.J. and Cohen, F.E. (1998) Prion protein biology. Cell, 93, 337-348.
Rao, C.N., Castronovo, V., Schmitt, M.C., Wewer, U.M., Claysmith, A.P., Liotta, L.A. and Sobel, M.E. (1989) Evidence for a precursor of the high-affinity metastasis-associated murine laminin receptor. Biochemistry, 28, 7476-7486.
Rao, N.C., Barsky, S.H., Terranova, V.P. and Liotta, L.A. (1983) Isolation of a tumor cell laminin receptor. Biochem. Biophys. Res. Commun., 111, 804-808.
Rieger, R., Edenhofer, F., Lasmezas, C.I. and Weiss, S. (1997) The human 37-kDa laminin receptor precursor interacts with the prion protein in eukaryotic cells. Nat Med, 3, 1383-1388. Rieger, R., Lasmezas, C.I. and Weiss, S. (1999) Role of the 37 kDa laminin receptor precursor in the life cycle of prions. Transfus Clin Biol, 6, 7-16.
Rohrer, J.W., Barsoum, A.L. and Coggin, J.H., Jr. (2001) The Development of a New Universal Tumor Rejection Antigen expressed on Human and Rodent Cancers for Vaccination, Prevention of Cancer, and Anti-Tumor Therapy. Mod Asp Immunobiol, 1, 191-195.
Rosenthal, E.T. and Wordeman, L. (1995) A protein similar tö the 67 kDa laminin binding protein and p40 is probably a component of the translational machinery in Urechis caupo oocytes and embryos. J. Cell. Sci., 108, 245-256.
Salas, P.J., Ponce, M.I., Brignoni, M. and Rodriguez, M.L. (1992) Attachment of Madin-Darby canine kidney cells to extracellular matrix: role of a laminin binding protein related to the 37/67 kDa laminin receptor in the development of plasma membrane polarization. Biol Cell, 75, 197-210.
Sato, M., Kinoshita, K., Kaneda, Y., Saeki, Y., Iwamatsu, A. and Tanaka, K. (1996) Analysis of nuclear localization of laminin binding protein precursor p40 (LBP/p40). Biochem Biophys Res Commun, 229, 896-901.
Sato, M., Saeki, Y., Tanaka, K. and Kaneda, Y. (1999) Ribosome-associated protein LBP/p40 binds to S21 protein of 40S ribosome: analysis using a yeast two-hybrid system. Biochem Biophys Res Commun, 256, 385-390.
Stephenson, D.A., Chiotti, K., Ebeling, C., Groth, D., DeArmond, S.J., Prusiner, S.B. and Carlson, G.A. (2000) Quantitative trait loci affecting prion incubation time in mice. Genomics, 69, 47-53.
Wang, K.S., Kuhn, R.J., Strauss, E.G., Ou, S. and Strauss, J.H. (1992) High-affinity laminin receptor is a receptor for Sindbis virus in mammalian cells. J Virol, 66, 4992-5001.
Wewer, U.M., Liotta, L., Jaye, M., Ricca, G.A., Drohan, W.N., Claysmith, A.P., Rao, C.N., Wirth, P., Coligan, J.E., Albrechtsen, R., Mudry, M. and Sobel, M.E. (1986) Altered levels of laminin receptor mRNA in various human carcinoma cells that have different abilities to bind laminin. Proc. Natl. Acad. Sci. USA, 83, 7137-71-41.
Yow, H., Wong, J.M., Chen, H.S., Lee, C., Steele, G.D.J. and Chen, L.B. (1988) Increased mRNA expression of a laminin-binding protein in human colon carcinoma: complete sequence of a full-length cDNA encoding the protein. Proc. Natl. Acad. Sci. USA, 85, 6394-6398.

### SEQUENZPROTOKOLL

(1) ALLGEMEINE ANGABEN:
Anmelder:

| | |
|---|---|
| Name: | Prof. Dr. Stefan Weiss |
| Strasse: | Elisabethstr. 30 |
| Ort: | München |
| Land: | Deutschland |
| Postleitzahl: | 80796 |

| | | |
|---|---|---|
| (A) | Name: | Prof. Dr. Melvyn Little |
| (B) | Strasse: | Fritz-von-Briesen-Str. 10 |
| (C) | Ort: | Neckargemünd |
| (D) | Land: | Deutschland |
| Postleitzahl: | | 69151 |

Erfinder:

| | | |
|---|---|---|
| (A) | Name: | Dr. Stefan Knackmuss |
| (B) | Strasse: | Uhlandstr. 13 |
| (C) | Ort: | Plankstadt |
| (D) | Land: | Deutschland |
| (E) | Postleitzahl: | 68723 |

| | | |
|---|---|---|
| (A) | Name: | Clemence Rey |
| (B) | Strasse: | Landsbergerstr. 111 |
| (C) | Ort: | München |
| (D) | Land: | Deutschland |
| (E) | Postleitzahl: | 80339 |

| | | |
|---|---|---|
| (A) | Name: | Claudia Büttner |
| (B) | Strasse: | Mittelgewann 42 |
| (C) | Ort: | Schwetzingen |
| (D) | Land: | Deutschland |
| (E) | Postleitzahl: | 68723 |

| | | |
|---|---|---|
| (A) | Name: | Dr. Peter Röttgen |
| (B) | Strasse: | Stahlbühlring 129 |
| (C) | Ort: | Ladenburg |
| (D) | Land: | Deutschland |
| (E) | Postleitzahl: | 68526 |

| | | |
|---|---|---|
| (A) | Name: | Dr. Uwe Reusch |
| (B) | Strasse: | Dieterwiesenstr. 13 |
| (C) | Ort: | Maikammer |
| (D) | Land: | Deutschland |
| (E) | Postleitzahl: | 67487 |

Single Chain Antikörper gegen den 37 kDa/67 kDa Lamininrezeptor als Werkzeuge zur Diagnose und Therapie von Prionerkrankungen, Herstellung und Verwendung
Zahl der Sequenzen: 4
Computerlesbare Fassung:
(A) Datenträger: CD
(B) Computer: IBM PC kompatibel
(C) Betriebssystem: PC-DOS/MS-DOS
(D) Software: WinWord 6.0

(2) INFORMATIONEN FÜR SEQ ID Nr. 1
(i)SEQUENZEIGENSCHAFTEN:
   (A) Länge: 816 Basenpaare
   (B) Art: Nucleinsäure
   (C) Strangform: Doppelstrang
   (D) Topologie: linear
(ii) MOLEKLÜLTYP: Nucleinsäure
   (A) Beschreibung: DNA kodiert für single chain Antikörper scFv S18. Die DNA ist im Plasmid pEX/HAM/LRP-S18 enthalten. Dieses Plasmid wurde bei der DSMZ, Mascheroder Weg 1b, D-38124 unter der Accesion Nummer xxxx hinterlegt. Nach Transformation des Plasmids in E.coli XL1 Blüe ist die Produktion des scFv Antikörpers S18 möglich.
(iii) Hypothetisch: nein
(iv) Antisense: nein
(xi) SEQL1ENZBESCHREIBLJNG: SEQ ID Nr. 1:

5' CAGG TGCAGCTGCA GGAGTCTGGG GGAGGCTTGG TACAGCCTGG
GGGGTCCCTG AGACTCTCCT GTGCAGCCTC TGGATTCATG TTTAGCAGGT
ATGCCATGAG CTGGGTCCGC CAGGCTCCAG GGAAGGGGCC AGAGTGGGTC
TCAGGTATTA GTGGTAGTGG TGGTAGTACA TACTACGCAG ACTCCGTGAA
GGGCCGGTTC ACCGTCTCCA GAGACAATTC CAAGAACACG CTGTATCTGC
AAATGAACAG CCTGAGAGCC GAGGACACGG CCGTATATTA CTGTGCGAGA
CATCCGGGTT TTTGGCATTT TGACTACTGG GGCCAGGGAA CTCTGGTCAC
CGTCTCCTCA GGGAGTGCAT CCGCCCCAAA GCTTGAAGAA GGTGAATTTT
CAGAAGCACG CGTATCTGAA CTGACTCAGG ACCCTGCTGT GTCTGTGGCC
TTGGGACAGA CAGTCAGGAT CACATGCCAA GGAGACAGCC TCAGAAACTT
TTATGCAAGC TGGTACCAGC AGAAGCCAGG ACAGGCCCCT ACTCTTGTCA
TCTATGGTTT AAGTAAAAGG CCCTCAGGGA TCCCAGACCG ATTCTCTGCC
TCCAGCTCAG GAAACACAGC TTCCTTGACC ATCACTGGGG CTCAGGCGGA
AGATGAGGCT GACTATTACT GTAACTCCCG GGACAGAAGT GGTAATCATG
TAAATGTGCT ATTCGGCGGA GGGACCAAGC TGACCGTCCT ACGTCAGCCC

AAGGCTGCCC CCTCGGTCAC TCTGTTCCCG CCCTCTTCTG CGGCCGCTGG
ATCCCATCAC CATCACCATC AC 3'

(2) INFORMATION FÜR SEQ ID Nr. 2
(i)SEQUENZEIGENSCHAFTEN:
   Länge: 268 Aminosäuren
   Art: Protein
   Strangform: Einzelstrang
   Topologie: linear
   MOLEKÜLTYP: Protein
   (A) Beschreibung: Dieses Protein entspricht dem single chain Antikörper S 18. Er kann nach Transformation des Plasmids pEx/HAM/LRP-S18 in E.coli XL1 Blue synthetisiert werden.
Hypothetisch: nein
Antisense: nein
(xi) SEQUENZBESCHREIBUNG: SEQ ID Nr. 2:

Amino-Terminus QVQLQESG GGLVQPGGSL RLSCAASGFM FSRYAMSWVR
QAPGKGPEWV SGISGSGGST YYADSVKGRF TVSRDNSKNT LYLQMNSLRA
EDTAVYYCAR HPGFWHFDYW GQGTLVTVSS GSASAPKLEE GEFSEARVSE
LTQDPAVSVA LGQTVRITCQ GDSLRNFYAS WYQQKPGQAP TLVIYGLSKR
PSGIPDRFSA SSSGNTASLT ITGAQAEDEA DYYCNSRDRS GNHVNVLFGG
GTKLTVLRQP KAAPSVTLFP PSSAAAGSHH HHHH Carboxy-Terminus

(2) INFORMATIONEN FÜR SEQ ID Nr. 3
(i)SEQUENZEIGENSCHAFTEN:
   (A) Länge: 834 Basenpaare
   (B) Art: Nucleinsäure
   (C) Strangform: Doppelstrang
   (D) Topologie:linear
(ii) MOLEKÜLTYP: Nucleinsäure
   (A) Beschreibung: DNA kodiert für single chain Antikörper scFv N3. Die DNA ist im Plasmid pEX/HAM/LRP-N3 enthalten. Dieses Plasmid wurde bei der DSMZ, Mascheroder Weg 1b, D-38124 unter der Accesion Nummer xxxx hinterlegt. Nach Transformation des Plasmids in E.coli KL1 Blue ist die Produktion des scFv Antikörpers N3 möglich.
(iii) Hypothetisch: nein
(iv) Antisense: nein
(xi) SEQUENZBESCHREIBUNG: SEQ ID Nr:3:

5' GAAG TGCAGCTGGT GGAGTCTGGG GGAGGCGTGG TCCAGCCTGG
GAGGTCCCTG AGACTCTCCT GTGCAGCGTC TGGATTCACC TTCAGTAGCT
ATGGCATGCA CTGGGTCCGC CAGGCTCCAG GCAAGGGGCT GGAGTGGGTG
GCAGTTATAT GGTATGATGG AAGTAATAAA TACTATGCAG ACTCCGTGAA
GGGCCGATTC ACCATCTCCA GAGACAATTC CAAGAACACG CTGTATCTGC
AAATGAACAG CCTGAGAGCC GAGGACACGG CTGTGTATTA CTGTGCGACT
ATACCGCGCT CGTCTTTCTA CTACGGTATG GACGTCTGGG GCCAAGGGAC
CACGGTCACC GTCTCCTCAG GGAGTGCATC CGCCCCAACC CTTAAGCTTG
AAGAAGGTGA ATTTTCAGAA GCACGCGTAC AGCCTGTGCT GACTCAGCCA
CCCTCAGCGT CTGGGACCCC AGGGCAGAGG GTCACCATCT CTTGTTCTGG
AAGCAGATCC AACATCGGAA GTAATACTGT AAACTGGTAC CAGCAGCTCC
CAGGAACGGC CCCCAAACTC CTCATCTATG GTAATAATCA GCGGCCCTCA
GGGGTCCCTG AGCGATTCTC TGGCTCCAAG TCTGGCACCT CAGCCTCCCT
GGCCATCAGT GGGCTCCAGT CAGAGGATGA GGCTGATTAT TACTGTGCAG
CGTGGGATGA CAGCCTGACT GGTGTGCTTT TCGGCGGAGG GACCAAGCTG

ACCGTCCTAG GTCAGCCCAA GGCTGCCCCC TCGGTCACTC TGTTCCCGCC
CTCTTCTGCG GCCGCTGGAT CCCATCACCA TCACCATCAC 3'

(2) INFORMATION FÜR SEQ ID Nr. 4
(i)SEQUENZEIGENSCHAFTEN:
   Länge: 278 Aminosäuren
   Art: Protein
   Strangform: Einzelstrang
   Topologie: linear
   MOLEKÜLTYP: Protein
   (A) Beschreibung: Dieses Protein entspricht dem single chain Antikörper N3. Er kann nach Transformation des Plasmids pEX/HAM/LRP-N3 in E.coli XL1 Blue synthetisiert werden.
   Hypothetisch: nein
(iv) Antisense: nein
(xi) SEQUENZBESCHREIBUNG: SEQ ID Nr. 4:

Aminoterminus EVQLVESGGG VVQPGRSLRL SCAASGFTFS SYGMHWVRQA
PGKGLEWVAV IWYDGSNKYY ADSVKGRFTI SRDNSKNTLY LQMNSLRAED
TAVYYCATIP RSSFYYGMDV WGQGTTVTVS SGSASAPTLK LEEGEFSEAR
VQPVLTQPPS ASGTPGQRVT ISCSGSRSNI GSNTVNWYQQ LPGTAPKLLI
YGNNQRPSGV PERFSGSKSG TSASLAISGL QSEDEADYYC AAWDDSLTGV
LFGGGTKLTV LGQPKAAPSV TLFPPSSAAA GSHHHHHH Carboxyterminus

## Patentansprüche

1. single-chain Anticörpermolekül, welches spezifisch gegen LRP/LR gerichtet ist und das die Aminosäuresequenz SEQ ID Nr. 2 umfasst.

2. Single-chain-Antikörpermolekül nach Anspruch 1, das die Aminosäuresequenz SEQ ID Nr. 2 besitzt.

3. cDNA, welche die Nukleotidsequenz SEQ ID Nr.1 umfasst und für ein Single-chain Antikörper, welcher spezifisch gegen LRP/LR gerichtet ist kodiert.

4. cDNA, welche die Nukleotidsequenz SEQ ID Nr. 1 aufweist und für ein Single-chain Antikörper, welcher spezifisch gegen LRP/LR gerichtet ist kodiert.

5. Single-chain-Antikörpermolekül, welches spezifisch gegen LRP/LR gerichtet ist und das die Aminosäuresequenz SEQ ID Nr. 4 umfasst.

6. Single-chain-Antikörpermolekül nach Anspruch 5, das die Aminosäuresequenz SEQ ID Nr. 4 besitzt.

7. cDNA, welche die Nukleotidsequenz SEQID Nr. 3 umfasst und für ein Single-chain Antikörper, welcher spezifisch gegen LRP/LR gerichtet ist kodiert.

8. cDNA, welche die Nukleotidsequenz SEQID Nr. 3 aufweist und für ein Single-chain Antikörper, welcher spezifisch gegen LRP/LR gerichtet ist kodiert.

9. Antikörpermolekül nach einem der Ansprüche 1 oder 5, das durch eine Aminosäureinsertion modifiziert ist, wobei es sich bei der Aminosäureinsertion um einen c-myc Tag handelt, welcher zwischen der F_{L}-Domäne und dem Hexahistidin Tag inseriert ist.

10. cDNA nach einem der Ansprüche 3,4, 7 oder 8, welche der Sequenz auf cDNA Ebene der modifizierten Antikörpermoleküle nach Anspruch 9 entspricht.

11. Antiköpermolekül nach einem der Ansprüche 1, 2, 5, 6 oder 9, das durch posstranslationale Modifikation verändert ist, wobei die posttranslationale Modifikation eine Glykosylierung, Phosphorylierung, Amidierung und/oder Acylierung darstellt.

12. Replikations- oder Expressionsvektor, der eine cDNA gemäss einem der Ansprüche 3, 4, 7, 8 oder 10 trägt.

13. Vektor nach Anspruch 12, wobei es sich um rekombinante Adeno-Assoziierte Viren (AAV) handelt.

14. Wirtszelle, die mit einem Replikations- oder Expressionsvektor nach Anspruch 12 oder 13 transformiert ist.

15. Wirtszelle nach Anspruch 14, wobei es sich um eine Säugerzelle handelt.

16. Wirtszelle nach Anspruch 15, wobei es sich um Muskelzellen des Typs C2.7 handelt.

17. Wirtszelle nach Anspruch 15, wobei es sich um Baby-Hamster Kidney Zellen handelt.

18. Wirtszelle nach Anspruch 15, wobei es sich um neuronale Zellen des Typs PC12 handelt.

19. Wirtszelle nach Anspruch 15, wobei es sich um neuronale Zellen des Typs N2a handelt.

20. Wirtszelle nach Anspruch 15, wobei es sich um neuronale Zellen des Typs GT1 handelt.

21. Wirtszelle nach Anspruch 15, wobei es sich um NIH3T3 Zellen handelt.

22. Verfahren zur Herstellung eines Antikörpermoleküls nach einem der Ansprüche 1, 2, 5 oder 6, das ein Kultivieren von Wirtszellen gemäss Anspruch 13 unter zur Expression eines erfindungsgemässen Antikörpermoleküls wirksamen Bedingungen umfasst.

23. Pharmazeutische Zusammensetzung, die ein Antikörpermolekül nach einem der Ansprüche 1, 2, 5 oder 6 in Verbindung mit einem pharmazeutisch akzeptablen Verdünnungsmittel und/oder Träger umfasst.

24. Pharmazeutische Zusammensetzung nach Anspruch 23, wobei sich die Zusammensetzung zur Behandlung von Prionerkrankungen eignet.

25. Diagnostische Zusammensetzung, die ein erfindungsgemässes Antikörpermolekül nach einem der Ansprüche 1, 2, 5 oder 6 in Verbindung mit einem akzeptablen Verdünnungsmittel und/oder Träger umfasst.

26. Diagnostische Zusammensetzung nach Anspruch 25, die zum Nachweis in Körperflüssigkeiten geeignet ist.

27. Diagnostische Zusammensetzung nach Anspruch 25, wobei es sich bei den Körperflüssigkeiten um Blut oder Cerebrospinalflüssigkeit (Liquor) handelt.

28. Diagnostische Zusammensetzung nach Anspruch 25, die zum Nachweis in Geweben geeignet ist.

29. Diagnostische Zusammensetzung nach Anspruch 28, wobei es sich bei den Geweben um Hirngewebe handelt.

30. Diagnostische Zusammensetzung nach Anspruch 28, wobei es sich bei den Geweben um lymphatisches Gewebe handelt.

31. Diagnostische Zusammensetzung nach Anspruch 25, die zum Nachweis von malignen Entartungen (Krebs) geeignet ist.

32. Diagnostische Zusammensetzung nach Anspruch 31, wobei der Nachweis in Körperflüssigkeiten erfolgt.

33. Diagnostische Zusammensetzung nach Anspruch 32, wobei es sich bei den Körperflüssigkeiten um Blut oder Liquor handelt.

34. Diagnostische Zusammensetzung nach Anspruch 31, wobei der Nachweis in Geweben erfolgt.

35. Verwendung eines Antikörpermoleküls nach einem der Ansprüche 1, 2, 5 oder 6 zur Herstellung eines Medikaments zur Behandlung einer Prionerkrankung oder Krebs.

## Claims

1. A single-chain antibody molecule specifically targeting LRP/LR and comprising the amino acid sequence SEQ ID NO: 2.

2. The single-chain antibody molecule according to claim 1, which has the amino acid sequence SEQ ID NO: 2.

3. A cDNA comprising the nucleotide sequence SEQ ID NO: 1 and encoding a single chain antibody that specifically targets LRP/LR.

4. A cDNA having the nucleotide sequence SEQ ID NO: 1 and encoding a single chain antibody that specifically targets LRP/LR.

5. A single-chain antibody molecule specifically targeting LRP/LR and comprising the amino acid sequence SEQ ID NO: 4.

6. The single-chain antibody molecule according to claim 5, which has the amino acid sequence SEQ ID NO: 4.

7. A cDNA comprising the nucleotide sequence SEQ ID NO: 3 and encoding a single chain antibody that specifically targets LRP/LR.

8. A cDNA having the nucleotide sequence SEQ ID NO: 3 and encoding a single chain antibody that specifically targets LRP/LR.

9. The antibody molecule according to any of claims 1 or 5, which is modified by an insertion of an amino acid, wherein said insertion of said amino acid is a c-myc tag that is inserted between the F_{L} domain and the hexahistidine tag.

10. The cDNA according to any of claims 3, 4, 7 or 8, which corresponds to the sequence at the cDNA level of said modified antibody molecules according to claim 9.

11. The antibody molecule according to any of claims 1, 2, 5, 6 or 9, which is modified by post-translational modification, wherein said post-translational modification is a glycosylation, phoshyorylation, amidation and/or acylation.

12. A replication or expression vector carrying a cDNA according to any of claims 3, 4, 7, 8 or 10.

13. The vector according to claim 12, wherein said vector are recombinant adeno-associated viruses (AAV).

14. A host cell being transformed with a replication or expression vector according to claim 12 or 13.

15. The host cell according to claim 14, wherein said host cell is a mammalian cell.

16. The host cell according to claim 15, wherein said host cells are muscle cells of the type C2.7.

17. The host cell according to claim 15, wherein said host cells are Baby-Hamster-Kidney cells.

18. The host cell according to claim 15, wherein said host cells are neuronal cells of the type PC 12.

19. The host cell according to claim 15, wherein said host cells are neuronal cells of the type N2a.

20. The host cell according to claim 15, wherein said host cells are neuronal cells of the type GT1.

21. The host cell according to claim 15, wherein said host cells are NIH3T3 cells.

22. A process for the production of an antibody molecule according to any of claims 1, 2, 5 or 6 comprising the cultivation of host cells according to claim 13 under conditions effective for the expression of an antibody molecule according to the invention.

23. A pharmaceutical composition comprising an antibody molecule according to any claims 1, 2, 5 or 6 in combination with a pharmaceutically acceptable diluent and/or vehicle.

24. The pharmaceutical composition according to claim 23, wherein said composition is suitable for the treatment of prion diseases.

25. A diagnostic composition comprising an antibody molecule according to the invention of any of claims 1, 2, 5 or 6 in combination with a pharmaceutically acceptable diluent and/or vehicle.

26. The diagnostic composition according to claim 25, which is suitable for the detection in body fluids.

27. The diagnostic composition according to claim 25, wherein said body fluids are blood or cerebrospinal fluid (liquor).

28. The diagnostic composition according to claim 25, which is suitable for the detection in tissues.

29. The diagnostic composition according to claim 28, wherein said tissues are brain tissue.

30. The diagnostic composition according to claim 28, wherein said tissues are lymphatic tissue.

31. The diagnostic composition according to claim 25, which is suitable for the detection of malignant degenerations (cancer).

32. The diagnostic composition according to claim 31, wherein said detection is carried out in body fluids.

33. The diagnostic composition according to claim 32, wherein said body fluids are blood or liquor.

34. The diagnostic composition according to claim 31, wherein said detection is carried out in tissues.

35. Use of an antibody molecule according to any of claims 1, 2, 5 or 6 for the manufacture of a medicament for the treatment of a prion disease or cancer.

## Revendications

1. Molécule anticorps à chaîne simple agissant spécifiquement contre LRP/LR et comprenant la séquence d'acides aminés SEQ ID N°2.

2. Molécule anticorps à chaîne simple selon la revendication 1, qui possède la séquence d'acides aminés SEQ ID N° 2.

3. cADN, qui comprend la séquence de nucléotides SEQ ID N° 1 et qui est codé pour un anticorps à chaîne simple agissant spécifiquement contre LRP/LR.

4. cADN, qui présente la séquence de nucléotides SEQ ID N° 1 et qui est codé pour un anticorps à chaîne simple agissant spécifiquement contre LRP/LR.

5. Molécule anticorps à chaîne simple, agissant spécifiquement contre LRP/LR et comprenant la séquence d'acides aminés SEQ ID N° 4.

6. Molécule anticorps à chaîne simple selon la revendication 5, qui possède la séquence d'acides aminés SEQ ID N°4.

7. cADN, qui comprend la séquence de nucléotides SEQ ID N° 3 et qui est codé pour un anticorps à chaîne simple agissant spécifiquement contre LRP/LR.

8. cADN, qui présente la séquence de nucléotides SEQ ID N° 3 et qui est codé pour un anticorps à chaîne simple agissant spécifiquement contre LRP/LR.

9. Molécule anticorps selon l'une des revendications 1 ou 5, qui est modifiée par une insertion d'acide aminé, dans laquelle il s'agit, pour l'insertion d'acide aminé, d'une étiquette c-myc qui est insérée entre les domaines, F_{L} et l'étiquette d'hexahistidine.

10. cDNA selon l'une des revendications 3, 4, 7 ou 8, qui correspond à la séquence au niveau de cADN de la molécule anticorps modifiée selon la revendication 9.

11. Molécule anticorps selon l'une des revendications 1, 2, 5, 6 ou 9, qui est modifiée par modification post-translationnelle, la modification post-translationnelle représentant une glycosylation, une phosphorylation, une amidation et/ou une acylation.

12. Vecteur de réplication ou d'expression, qui porte un cADN selon l'une des revendications 3, 4, 7, 8 ou 10.

13. Vecteur selon la revendication 12, dans lequel il s'agit de virus adéno-associés recombinants (VAA).

14. Cellule hôte, qui est transformée par un vecteur de réplication ou d'expression selon la revendication 12 ou 13.

15. Cellule hôte selon la revendication 14, dans laquelle il s'agit d'une cellule de mammifère.

16. Cellule hôte selon la revendication 15, dans laquelle il s'agit de cellules de muscles du type C2.7.

17. Cellule hôte selon la revendication 15, dans laquelle il s'agit de cellules de 'Baby-Hamster Kidney' (= de reins de jeunes hamsters).

18. Cellule hôte selon la revendication 15, dans laquelle il s'agit de cellules neuronales du type PC12.

19. Cellule hôte selon la revendication 15, dans laquelle il s'agit de cellules neuronales du type N2a.

20. Cellule hôte selon la revendication 15, dans laquelle il s'agit de cellules neuronales du type GT1.

21. Cellule hôte selon la revendication 15, dans laquelle il s'agit de cellules NIH3T3.

22. Procédé pour produire une molécule anticorps selon l'une des revendications 1, 2, 5 ou 6, qui comprend le fait de cultiver des cellules hôtes selon la revendication 13 dans des conditions actives pour l'expression d'une molécule anticorps selon l'invention.

23. Composition pharmaceutique, qui comprend une molécule anticorps selon l'une des revendications 1, 2, 5 ou 6 en combinaison avec un diluant pharmaceutiquement acceptable et/ou un porteur.

24. Composition pharmaceutique selon la revendication 23, dans laquelle la composition convient pour le traitement de maladies à prions.

25. Composition diagnostique, qui comprend une molécule anticorps selon l'une des revendications 1, 2, 5 ou 6 en combinaison avec un diluant acceptable et/ou un porteur.

26. Composition diagnostique selon la revendication 25, qui convient pour la détection dans des liquides du corps.

27. Composition diagnostique selon la revendication 25, dans laquelle il s'agit, pour les liquides du corps, de sang ou de liquide cérébro-spinal.

28. Composition diagnostique selon la revendication 25, qui convient pour la détection dans des tissus.

29. Composition diagnostique selon la revendication 28, dans laquelle il s'agit, pour les tissus, de tissu cérébral.

30. Composition diagnostique selon la revendication 28, dans laquelle il s'agit, pour les tissus, de tissu lymphatique.

31. Composition diagnostique selon la revendication 25, qui convient pour la détection de cancérisations (cancer).

32. Composition diagnostique selon la revendication 31, dans laquelle la détection a lieu dans des liquides du corps.

33. Composition diagnostique selon la revendication 32, dans laquelle il s'agit, pour les liquides du corps, de sang ou de liqueur.

34. Composition diagnostique selon la revendication 31, dans laquelle la détection a lieu dans des tissus.

35. Utilisation d'une molécule anticorps selon l'une des revendications 1, 2, 5 ou 6 pour la préparation d'un médicament pour le traitement d'une maladie à prions ou d'un cancer.
